(19) Europäisches Patentamt
European Patent Office
Office européen des brevets

(11) EP 1 466 892 A1

(12) EUROPÄISCHE PATENTANMELDUNG

(43) Veröffentlichungstag:
**13.10.2004 Patentblatt 2004/42**

(21) Anmeldenummer: **04102032.2**

(22) Anmeldetag: **30.10.2001**

(51) Int Cl.[7]: **C07C 255/40**, C07D 319/18, C07F 7/08, G03C 1/815, A61K 7/42

(84) Benannte Vertragsstaaten:
**AT BE CH CY DE DK ES FI FR GB GR IE IT LI LU MC NL PT SE TR**

(30) Priorität: **10.11.2000 DE 10055940**

(62) Dokumentnummer(n) der früheren Anmeldung(en) nach Art. 76 EPÜ:
**01993598.0 / 1 341 752**

(71) Anmelder: **Symrise GmbH & Co. KG**
**37603 Holzminden (DE)**

(72) Erfinder:
- **Koch, Oskar**
  **37079, Göttingen (DE)**
- **Dilk, Erich**
  **37603, Holzminden (DE)**
- **Langner, Roland**
  **37639, Bevern (DE)**
- **Johncock, William**
  **37671, Höxter (DE)**

(74) Vertreter: **Eisenführ, Speiser & Partner**
**Patentanwälte Rechtsanwälte**
**Postfach 10 60 78**
**28060 Bremen (DE)**

Bemerkungen:
Diese Anmeldung ist am 11 - 05 - 2004 als Teilanmeldung zu der unter INID-Kode 62 erwähnten Anmeldung eingereicht worden.

(54) **Neue Indanylidenverbindungen**

(57) Neue Indanylidenverbindungen können als UV-A-Filter in kosmetischen Mitteln zum Schutz von Haut und Haaren und für technische Anwendungen verwendet werden.

EP 1 466 892 A1

## Beschreibung

Die Erfindung betrifft neue Indanylidenverbindungen, ein Verfahren zu ihrer Herstellung und ihre Verwendung als UVA-Filter.

Indanylidenverbindungen, die UV-absorbierende Eigenschaften haben, sind aus der EP-A 823 418 bereits bekannt. Die in der EP-A 823 418 vorbeschriebenen Indanylidenverbindungen haben jedoch eine für die Anwendung zu geringe Photostabilität.

Es wurden neue Indanylidenverbindungen der Formel

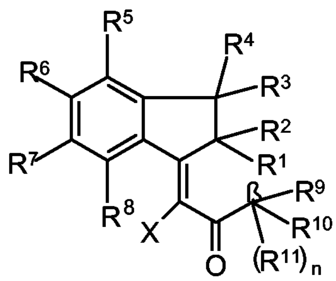

(I)

worin

| | |
|---|---|
| $R^1$ bis $R^4$ | unabhängig voneinander Wasserstoff, $C_1$-$C_{20}$-Alkyl oder $C_5$-$C_{10}$-Cycloalkyl bedeuten, mit der Maßgabe, dass auch zwei Substituenten an benachbarten C-Atomen zusammen eine gegebenenfalls substituierte $C_1$-$C_4$-Alkylengruppe bedeuten können;<br>weiterhin unabhängig voneinander $C_2$-$C_{20}$-Alkyl, in der mindestens eine Methylengruppe durch Sauerstoff, $C_3$-$C_{20}$-Alkenyl, $C_3$-$C_{20}$-Alkinyl oder einer Gruppe S ersetzt sein kann, bedeuten können,<br>wobei S eine Silan-, eine Oligosiloxan- oder eine Polysiloxan-Gruppe sein kann; |
| $R^5$ bis $R^8$ | unabhängig voneinander Wasserstoff, $C_1$-$C_{20}$-Alkyl oder $C_5$-$C_{10}$-Cycloalkyl oder $C_1$ -$C_{20}$-Alkoxy, $C_5$-$C_{10}$-Cycloalkoxy, Hydroxy, Acetoxy, Acetamino, Carboxy, Carbalkoxy oder Carbamoyl bedeuten, zusätzlich zwei Substituenten von $R^5$ bis $R^8$ an benachbarten C-Atomen zusammen einen 5-7-gliedrigen Ring bilden können, der bis zu drei Heteroatome, insbesondere Sauerstoff oder Stickstoff enthält, wobei die Ringatome durch exocyclisch doppelt gebundenen Sauerstoff (Ketogruppe) substituiert sein können, weiterhin im Fall von Alkoxy unabhängig voneinander $C_2$-$C_{20}$-Alkyl, in der mindestens eine Methylengruppe durch Sauerstoff, $C_3$-$C_{20}$-Alkenyl, $C_3$-$C_{20}$ Alkinyl oder einer Gruppe S ersetzt sein kann, bedeuten können,<br>wobei S eine Silan-, eine Oligosiloxan- oder eine Polysiloxan-Gruppe sein kann; |
| X | für Cyano, $CON(R)_2$ oder $CO_2R$ wobei R für Wasserstoff oder $C_1$-$C_8$-Alkyl steht; |
| n | für 1 oder 0 steht; |
| $R^9$ bis $R^{11}$ | im Fall von n=1 Wasserstoff, $C_1$-$C_{20}$-Alkyl oder $C_5$-$C_{10}$-Cycloalkyl, Aryl oder Heteroaryl bedeuten können, zusätzlich zwei Substituenten von $R^9$ bis $R^{11}$ gemeinsam mit dem β-Atom einen 3-7-gliedrigen Ring, der bis zu drei Heteroatome, insbesondere Sauerstoff oder Stickstoff enthalten kann, bilden können,<br>weiterhin im Fall von n=0 $R^9$ und $R^{10}$ gemeinsam mit dem β-Atom einen Aryl oder Heteroaryl-Rest bedeuten, |

gefunden.

Die neuen Indanylidenverbindungen stellen eine überraschende Auswahl unter den aus der EP-A 823 418 bekannten Indanylidenverbindungen dar. Sie haben eine deutlich höhere Photostabilität als die in EP-A 823 418 genannten Verbindungen und eine hohe Kompatibilität mit anderen UV-Filtern, wie z.B. p-Methoxyzimtsäure-isooctylester.

Bevorzugt sind Indanylidenverbindungen der Formel

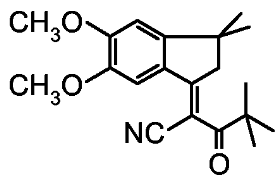
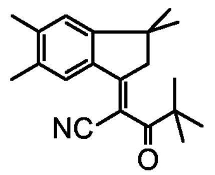
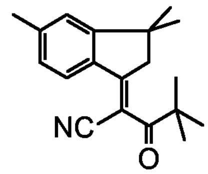

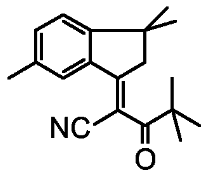
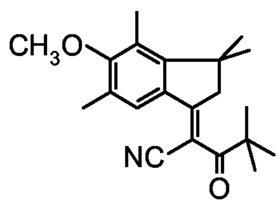
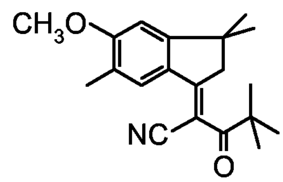

Insbesondere bevorzugt sind Indanylidenverbindungen der Formel

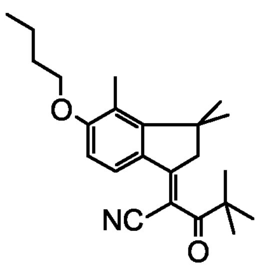
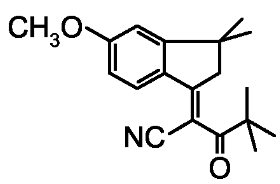
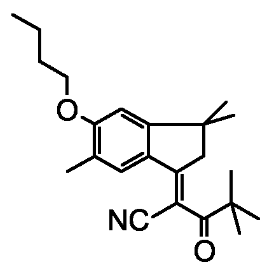
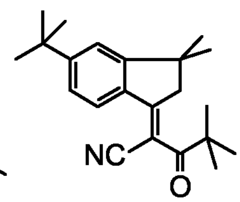

Im Einzelnen seien die folgenden bevorzugten Indanylidenverbindungen genannt: 2-(5,6-Dimethoxy-3,3-dimethyl-1-indanyliden)-4,4-dimethyl-3-oxo-pentansäurenitril, 2-(5-Methoxy-3,3,4,6-tetramethyl-1-indanyliden)-4,4-dimethyl-3-oxo-pentansäurenitril, 2-(3,3,5,6-tetramethyl-1-indanyliden)-4,4-dimethyl-3-oxo-pentansäurenitril, 2-(3,3,6-trimethyl-1-indanyliden)-4,4-dimethyl-3-oxo-pentansäurenitril, 2-(5,6-Ethylendioxo-3,3-dimethyl-1-indanyliden)-4,4-dimethyl-3-oxo-pentansäurenitril, 2-(5-Methoxy-3,3,6-trimethyl-1-indanyliden)-4,4-dimethyl-3-oxo-pentansäurenitril, 2-[(5-Methoxy-3,3-dimethyl-(2-methyl-3-(1,3,3,3-tetramethyl-1-(trimethyl-silyloxy)-disiloxanyl)-propyl)-indanyliden)]-4,4-dimethyl-3-oxo-pentansäurenitril und 2-(6-Acetoxy-3,3-dimethyl-5-methoxy-1-indanyliden)-4,4-dimethyl-3-oxo-pentansäurenitril.

Die erfindungsgemäßen Indanylidenverbindungen können durch (Knoevenagel)-Kondensation von Verbindungen der Formel

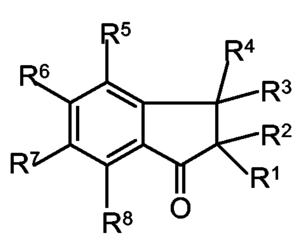
(II)

wobei
$R^1$ bis $R^8$ die oben genannte Bedeutung haben,
mit Verbindungen der Formel

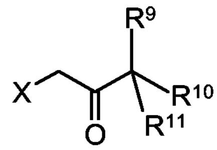

(III)

wobei
$R^9$ bis $R^{11}$ und X die oben genannte Bedeutung haben,
hergestellt werden.

Die hierfür verwendeten Indanone können durch Friedel-Crafts-Reaktionen von (substituierten) Acrylsäureestern mit (substituierten) Aromaten oder im Fall von Hydroxysubstituenten durch Fries'sche Umlagerung entsprechender Phenolester hergestellt werden (F.-H. Marquardt, Helv. Chim. Acta 159, 1476 (1965)).

Die Herstellung der erfindungsgemäßen Indanylidenverbindungen kann beispielsweise wie folgt durchgeführt werden:

Man kondensiert die o.a. Indanone mit eqimolaren Mengen Pivaloylacetonitril unter der Katalyse von Ammoniumacetat gemäß den Bedingungen einer Knoevenagel-Kondensation.

Die Herstellung kann durch das folgende Reaktionsschema erläutert werden:

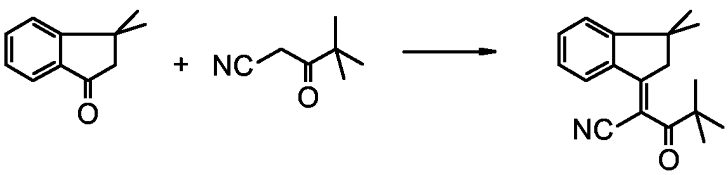

Die erfindungsgemäßen Indanylidenverbindungen können als UV-Absorber, z.B. in kosmetischen Mitteln, insbesondere zum Schutz vor akuten (Sonnenbrand) sowie chronischen (frühzeitiger Hautalterung) Hautschäden besonders in Sonnenschutzmitteln, Tagespflegeprodukten und Haarpflegeprodukten, aber auch zur Verbesserung der Lichtbeständigkeit von technischen Produkten, wie Anstrichen, Lacken, Kunststoffe, Textilien, Verpackungsmaterialien und Kautschuken verwendet werden.

Die erfindungsgemäßen Indanylidenverbindungen können einzeln oder in Mischung in den entsprechenden Zubereitungen eingesetzt werden; man kann sie auch in Kombination mit UV-Absorbern anderer Substanzklassen oder auch mit diesen in beliebigen Gemischen untereinander einsetzen. Beispielsweise seien die folgenden UV-Absorber genannt:

- p-Aminobenzoesäure
- p-Aminobenzoesäureethylester (25 Mol) ethoxyliert
- p-Dimethylaminobenzoesäure-2-ethylhexylester
- p-Aminobenzoesäureethylester (2 Mol) N-propoxyliert
- p-Aminobenzoesäureglycerinester
- Salicylsäure-homomenthylester (Homosalate) (Neo Heliopan® HMS)
- Salicylsäure-2-ethylhexylester (Neo Heliopan® OS)
- Triethanolaminsalicylat
- 4-Isopropylbenzylsalicylat
- Anthranilsäurementhylester (Neo Heliopan® MA)
- Diisopropylzimtsäureethylester
- p-Methoxyzimtsäure-2-ethylhexylester (Neo Heliopan® AV)
- Diisopropylzimtsäuremethylester
- p-Methoxyzimtsäureisoamylester (Neo Heliopan® E 1000)
- p-Methoxyzimtsäure-diethanolaminsalz
- p-Methoxyzimtsäure-isopropylester
- 2-Ethylhexyl-2-cyano-3,3-diphenylacrylat (Neo Heliopan® 303)
- Ethyl-2-cyano-3,3'-diphenylacrylat
- 2-Phenylbenzimidazolsulfonsäure und Salze (Neo Heliopan® Hydro)

- 3-(4'-Trimethylammonium)-benzyliden-bornan-2-on-methylsulfat
- Terephthalyliden-dibornansulfonsäure und Salze (Mexoryl® SX)
- 4-t-Butyl-4'-methoxy-dibenzoylmethan (Avobenzon) / (Neo Heliopan® 357)
- ß-Imidazol-4(5)-acrylsäure (Urocaninsäure)
- 2-Hydroxy-4-methoxybenzophenon (Neo Heliopan® BB)
- 2-Hydroxy-4-methoxybenzophenon-5-sulfonsäure
- Dihydroxy-4-methoxybenzophenon
- 2,4-Dihydroxybenzophenon
- Tetrahydroxybenzophenon
- 2,2'-Dihydroxy-4,4'-dimethoxybenzophenon
- 2-Hydroxy-4-n-octoxybenzophenon
- 2-Hydroxy-4-methoxy-4'-methylbenzophenon
- 3-(4'-Sulfo)benzyliden-bornan-2-on und Salze
- 3-(4'-Methylbenzyliden)-d,1-campher (Neo Heliopan® MBC)
- 3-Benzyliden-d,1-campher
- 4-Isopropyldibenzoylmethan
- 2,4,6-Trianilino-(p-carbo-2'-ethylhexyl-1'-oxy)-1,3,5-triazin
- Phenylen-bis-benzimidazyl-tetrasulfonsäure-dinatriumsalz (Neo Heliopan® AP)
- 2,2'-(1,4-Phenylen)-bis-(1H-benzimidazol-4,6-disulfonsäure), Mononatriumsalz
- N-[(2 und 4)-[2-(oxoborn-3-yliden)methyl]benzyl]-acrylamid-Polymer
- Phenol, 2-(2H-benzotriazol-2-yl)-4-methyl-6-(2-methyl-3(1,3,3,3-tetramethyl-1-(trimethylsilyl)-oxy)-disiloxyanyl)-propyl), (Mexoryl® XL)
- 4,4'-[(6-[4-(1,1-Dimethyl)-aminocarbonyl)-phenylamino]-1,3,5-triazin-2,4-diyl)diimino] -bis-(benzoesäure-2-ethylhexylester) (Uvasorb® HEB)
- 2,2'-Methylen-bis-(6-(2H-benztriazol-2-yl)-4-1,1,3,3-tetramethylbutyl)-phenol), (Tinosorb® M)
- 2,4-bis-[4-(2-ethylhexyloxy)-2-hydroxyphenyl]-1,3,5-triazin
- Benzylidenmalonat-Polysiloxan (Parsol® SLX)
- Glyceryl-ethylhexanoat-dimethoxycinnamat
- Dinatrium-2,2'-dihydroxy-4,4'-dimethoxy-5,5'-disulfo-benzophenon
- Dipropylenglykolsalicylat
- Natrium-hydroxymethoxybenzophenon-sulfonat
- 4,4',4-(1,3,5-Triazin-2,4,6-triyltriimino)-tris-benzoesäure-tris(2-ethylhexylester) (Uvinul® T150)
- 2,4-Bis-[{(4-(2-Ethyl-hexyloxy)-2-hydroxy}-phenyl]-6-(4-methoxyphenyl)-1,3,5-triazin, (Tinosorb® S)
- 2,4-Bis-[{(4-(3-sulfonato)-2-hydroxy-propyloxy)-2-hydroxy}-phenyl]-6-(4-methoxyphenyl)-1,3,5-triazin-Natriumsalz
- 2,4-Bis-[{(3-(2-Propyloxy)-2-hydroxy-propyloxy)-2-hydroxy}-phenyl]-6-(4-methoxy-phenyl)-1,3,5-triazin
- 2,4-Bis-[{4-(2-Ethyl-hexyloxy)-2-hydroxy}-phenyl]-6-[4-(2-methoxyethylcarbonyl)-phenylamino]-1,3,5-triazin
- 2,4-Bis-[{4-(3-(2-propyloxy)-2-hydroxy-propyloxy)-2-hydroxy}-phenyl]-6-[4-(2-ethylcarboxyl)-phenylamino]-1,3,5-triazin
- 2,4-Bis-[{4-(2-Ethyl-hexyloxy)-2-hydroxy}-phenyl]-6-(1-methyl-pyrrol-2-yl-)-1,3,5-triazin
- 2,4-Bis-[{4-tris-(trimethylsiloxy-silylpropyloxy)-2-hydroxy}-phenyl]-6-(4-methoxyphenyl) - 1, 3,5 -triazin
- 2,4-Bis-[{4-(2"-Methylpropenyloxy)-2-hydroxy}-phenyl]-6-(4-methoxyphenyl)-1,3,5-triazin
- 2,4-Bis-[14-(1',1',1',3'5',5',5'-Heptamethylsiloxy-2"-methyl-propyloxy)-2-hydroxy} -phenyl] -6-(4-methoxyphenyl)-1, 3, 5-triazin

Besonders geeignete UV-Absorber sind:

- p-Aminobenzoesäure
- 3-(4'-Trimethylammonium)-benzyliden-bornan-2-on-methylsulfat
- Salicylsäure-homomenthylester (Neo Heliopan® HMS)
- 2-Hydroxy-4-methoxy-benzophenon (Neo Heliopan® BB)
- 2-Phenylbenzimidazolsulfonsäure (Neo Heliopan® Hydro)
- Terephthalyliden-dibornansulfonsäure und Salze (Mexoryl® SX)
- 4-tert.-Butyl-4'-methoxydibenzoylmethan (Neo Heliopan® 357)
- 3-(4'-Sulfo)benzyliden-bornan-2-on und Salze
- 2-Ethylhexyl-2-cyano-3,3-diphenylacrylat (Neo Heliopan® 303)
- N-[(2 und 4)-[2-(oxoborn-3-yliden)methyl]benzyl]-acrylamid-Polymer
- p-Methoxyzimtsäure-2-ethylhexylester (Neo Heliopan® AV)

- p-Aminobenzoesäure-ethylester (25 Mol) ethoxyliert
- p-Methoxyzimtsäure-isoamylester (Neo Heliopan® E1000)
- 2,4,6-Trianilino-(p-carbo-2'-ethylhexyl-1'-oxy)-1,3,5-triazin (Uvinul® T150)
- Phenol, 2-(2H-benzotriazol-2-yl)-4-methyl-6-(2-methyl-3(1,3,3,3-tetramethyl-1-(trimethylsilyl)-oxy)-disiloxyanyl)-propyl), (Mexoryl® XL)
- 4,4'-[(6-[4-(1,1-Dimethyl)-aminocarbonyl)-phenylamino]-1,3,5-triazin-2,4-diyl)-diimino]-bis-(benzoesäure-2-ethylhexylester), (UvasorbHEB)
- 3-(4'-Methylbenzyliden)-d,1-campher (Neo Helipan® MBC)
- 3-Benzylidencampher
- Salicylsäure-2-ethylhexylester (Neo Helipan® OS)
- 4-Dimethylaminobenzoesäure-2-ethylhexylester (Padimate O)
- Hydroxy-4-methoxy-benzophenon-5-sulfonsäure und Na-Salz
- 2,2'-Methylen-bis-(6-(2H-benztriazol-2-yl)-4-1,1,3,3-tetramethylbutyl)-phenol), (Tinosorb® M)
- Phenylen-bis-benzimidazyl-tetrasulfonsäure-dinatriumsalz (Neo Heliopan® AP)
- 2,4-Bis-[{(4-(2-Ethyl-hexyloxy)-2-hydroxy}-phenyl]-6-(4-methoxyphenyl)-1,3,5-triazin, (Tinosorb® S)
- Benzylidenmalonat-Polysiloxan (Parsol® SLX)
- Menthylanthranilat (Neo Heliopan® MA)

Es kann auch von Vorteil sein, polymer-gebundene oder polymere UV-Absorber in Zubereitungen gemäß der vorliegenden Erfindung zu verwenden, insbesondere solche, wie sie in WO-A-92/20690 beschrieben werden. Ebenso ist die Kombination der erfindungsgemäßen Indanylidenverbindungen mit feinteiligen anorganischen und organischen Pigmenten, wie z.B. Titandioxid, Zinkoxid und Eisenoxid bzw. Tinosorb® M, in Sonnenschutz- und Tagespflegeprodukten mit UV-Schutz möglich.

Die Aufzählung der genannten UV-Filter, die im Sinne der vorliegenden Erfindung eingesetzt werden können, soll selbstverständlich nicht limitierend sein.

Die Gesamtmenge aller (einfach und mehrfach sulfonierter) wasserlöslichen UV-Filtersubstanzen in den fertigen kosmetischen oder dermatologischen Zubereitungen, beispielsweise an Phenylen-bis-benzimidazyl-tetrasulfonsäure-dinatriumsalz bzw. deren Salzen und/oder die entsprechende Disulfonsäure bzw. deren Salzen und/oder 2-Phenylbenzimidazol-5-sulfonsäure bzw. deren Salzen und/oder 2-Hydroxy-4-methoxybenzophenon-5-sulfonsäure bzw. deren Salzen und/oder 4-(2-Oxo-3-bornylidenmethyl)-benzolsulfonsäure bzw. deren Salzen und/oder 2-Methyl-5-(2-oxo-3-bornyliden-methyl)-benzolsulfonsäure bzw. deren Salzen und/oder Benzol-1,4-di-(2-oxo-3-bornylidenmethyl)-10-sulfonsäure bzw. deren Salzen, wird vorteilhaft aus dem Bereich von 0,1 bis 10,0 Gew.-%, bevorzugt 0,5 bis 6,0 Gew.-% gewählt, bezogen auf das Gesamtgewicht der Zubereitungen, falls die Gegenwart dieser Substanzen erwünscht ist.

Die Gesamtmenge an öllöslichen UV-Filtersubstanzen in den fertigen kosmetischen oder dermatologischen Zubereitungen, beispielsweise an 4,4' ,4' '-( 1 ,3 ,5 -Triazin-2,4,6-triyltriimino)-tris-benzoesäure-tris-(2-ethylhexylester) und/oder 4-tert.-Butyl-4'methoxy-dibenzoylmethan und/oder 4-Methylbenzylidencampher und/oder Octyldimethyl-p-Aminobenzoesäure und/oder Mexoryl® XL und/oder Uvasorb® HEB und/oder Tinosorb® S und/oder Benzophenon-3 und/oder Parsol® SLX und/oder Neo Heliopan® MA wird vorteilhaft aus dem Bereich von 0,1 bis 10,0 Gew.-%, bevorzugt 0,5 bis 6,0 Gew.-% gewählt, bezogen auf das Gesamtgewicht der Zubereitungen, falls die Gegenwart dieser Substanzen erwünscht ist.

Die Gesamtmenge an 2-Ethylhexyl-p-methoxy-cinnamaten und/oder p-Methoxyzimtsäureisoamylester in den fertigen kosmetischen oder dermatologischen Zubereitungen wird vorteilhaft aus dem Bereich von 0,1 bis 15,0 Gew.-%, bevorzugt 0,5 bis 7,5 Gew.-% gewählt, bezogen auf das Gesamtgewicht der Zubereitungen, falls die Gegenwart dieser Substanzen erwünscht ist.

Die Gesamtmenge an Ethylhexyl-2-cyano-3,3-diphenylacrylat in den fertigen kosmetischen oder dermatologischen Zubereitungen wird, falls die Gegenwart dieser Substanz erwünscht ist, vorteilhaft aus dem Bereich von 0,1 bis 15,0 Gew.-%, bevorzugt 0,5 bis 10,0 Gew.-% gewählt, bezogen auf das Gesamtgewicht der Zubereitungen.

Die Gesamtmenge an einem oder mehreren Salicylsäurederivaten in den fertigen kosmetischen oder dermatologischen Zubereitungen wird vorteilhaft aus dem Be-reich von 0,1 bis 15,0 Gew.-%, bevorzugt 0,5 bis 10,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 bis 5,0 Gew.-% zu wählen. Wenn Homomenthylsalicylat gewählt wird, ist es von Vorteil, dessen Gesamt-menge aus dem Bereich von 0,1 bis 10,0 Gew.-% zu wählen.

Die erfindungsgemäßen Indanylidenverbindungen eignen sich auch in besonderem Maße zur Photostabilisierung von UV-Absorbern mit geringer UV-Lichtstabilität. Insbesondere gelingt die Photostabilisierung der sehr lichtinstabilen Verbindungen der Dibenzoylmethane, z.B. tert.-Butyl-4'-methoxydibenzoylmethan.

Eine weitere lichtstabile UV-Filterkombination wird erreicht durch Einsatz von 0,1 bis 10 Gew.-%, bevorzugt 1 bis 10 Gew.-% p-Methoxyzimtsäure-ethylhexylester oder - isoamylester mit 0,1 bis 10 Gew.-%, bevorzugt 1 bis 6

Gew.-% der Verbindung der Formel I, bevorzugt im Verhältnis 1:1.

Die Kombinationen von p-Methoxyzimtsäureestern und Dibenzoylmethanderivaten und Verbindungen der Formel I lassen sich lichtstabil formulieren durch Einsatz von z.B. 0,1 bis 5 Gew.-%, bevorzugt 1 bis 3 Gew.-% 4-tert. -Butyl-4'-methoxydibenzoylmethan, 0,1 bis 10 Gew.-%, bevorzugt 1 bis 7,5 Gew.-% p-Methoxyzimtsäureethylhexyl- oder isoamylester und mindestens 0,2 Gew.-%, bevorzugt 1 bis 6 Gew.-% der Verbindungen der Formel I, bevorzugt im Verhältnis 1 Teil Dibenzoylmethanderivat, 2 Teile p-Methoxyzimtsäureester und 2 Teile der erfindungsgemäßen Indanylidenverbindungen.

Vorteilhaft ist weiterhin zu dieser Dreierkombination einen oder weitere sehr photostabile UV-Absorber, wie z.B. Methylbenzylidencampher, 2-Ethylhexyl-2-cyano-3,3'diphenylacrylat, Octyltriazon, Uvasorb® HEB, Tinosorb® S, Tinosorb® M, Ethylhexylsalicylat, Homomenthylsalicylat sowie Phenylenbenzimidazolsulfonsäure oder Phenylen-bis-benzimidazol-tetrasulfonsäure-dinatriumsalz, Mexoryl® SX, Mexoryl® XL oder Parsol® SLX hinzuzusetzen.

Weiterhin wird in kosmetischen Zubereitungen überraschenderweise durch die Verwendung von Indanyliden-Derivaten der Formel I in Kombination mit anderen UV-Filtem eine synergistische Erhöhung des Sonnenschutzfaktors erreicht. Beispiele für eine synergistische Erhöhung des Sonnenschutzfaktors sind kosmetische Emul-sionen, die sowohl eine Verbindung der Formel I als auch Ethylhexylmethoxy-cinnamat oder Octocrylene, oder eine Kombination von Verbindung der Formel I mit Ethylhexylmethoxycinnamat und 2-Phenylbenzimidazolsulfonsäure, oder Ethylhexylmethoxycinnamat und Methylbenzylidencampher, oder Ethylhexyl-methoxycinnamat und 4-t-Butyl-4'-methoxy-dibenzoylmethan, oder Neo Heliopan® AP und Ethylhexylmethoxycinnamat, oder eine Kombination von Verbindung der Formel I mit Octocrylene, Methylbenzylidencampher und Zinkoxid enthalten. Ebenfalls weisen Kombinationen von Verbindung der Formel I mit Dibenzoylmethanen, Methylbenzylidencampher, 2-Phenylbenzimidazolsulfonsäure, Neo Heliopan® AP, Mexoryl® SX, Mexoryl ® XL, Parsol® SLX, Tinosorb® S, Tinosorb® M, Uvinul® T150, Uvasorb® HEB sowie mikrofeinen Pigmenten, Zinkoxid und Titandioxid, synergistische Erhöhungen des Sonnenschutzfaktors auf. Die genannten UV-Filter-Kombinationen sind beispielhaft angeführt und nicht auf die oben genannten Kombinationen beschränkt zu verstehen. So können alle auf Seiten 8/9 bereits genannten besonders geeigneten UV-Absorber sowie alle in den nachfolgenden Veröffentlichungen zugelassenen UV-Filter mit Verbindungen der Formel I oder o.a. Kombinationen, einzeln oder in beliebigen Mischungen, kombiniert eingesetzt werden.

| | |
|---|---|
| USA: | Food and Drug Administration (FDA). Veröffentlichung in Monographie für Sunscreen Drug Products for Over-The-Counter Human Use. |
| Europa: | Richtlinie 76/768 EWG des Rates zur Angleichung der Rechtsvorschriften der Mitgliedsstaaten über kosmetische Mittel an den technischen Fortschritt. Veröffentlichungen im Official Journal of European Communities. |
| Japan: | Veröffentlichung der Kosmetik-Richtlinie des Ministry of Health and Welfare (MHW). |
| Deutschland: | Veröffentlichung in der Verordnung über kosmetische Mittel gemäß dem Lebensmittel- und Bedarfsgegenstände-Gesetz (LMBG). |
| Australien: | Registrierung durch Therapeutic Goods Administration (TGA) und Veröffentlichung im Australian Register of Therapeutic Goods (ARTG). |

Üblicherweise werden durch diese Kombinationen eine synergistische Erhöhung des UV-Sonnenschutzfaktors erzielt.

Die Kombination von Verbindungen der Formel I mit UV-A-Absorbern, besonders UV-A-II-Absorbern, ergibt einen umfassenden Schutz gegen die UV-A-Strahlung (320-400 nm). Insbesondere eine Kombination von Verbindungen der Formel I mit Neo Heliopan® AP (UV-AII-Absorber) ist für eine breite UV-A-Schutzleistung zu nennen. Weitere UV-A-Filter, die in Kombination mit Verbindungen der Formel I allein oder in Kombination von Verbindungen der Formel I und Neo Heliopan® AP bevorzugt werden, sind Mexoryl® SX, Mexoryl® XL, Tinosorb® M Tinosorb® S, Benzophenon-3, Benzophenon-4, Neo Heliopan® 357, Neo Heliopan® MA.

Für eine optimale Breitbandschutzleistung gegen UV-A- und UV-B-Strahlung sind vorgenannte Kombinationen mit allen UV-B-Filtem und Mischungen aus diesen Filtern zu kombinieren (vgl. Liste auf Seiten 6-9). Bevorzugt geeignet sind Neo Heliopan® AV, Neo Heliopan® E1000, Neo Heliopan® Hydro, Neo Heliopan® MBC, Neo Heliopan® 303, Neo Heliopan® OS, Neo Heliopan® HMS, Uvinul® T150, Uvasorb® HEB, Dimethylaminobenzoesäure-ethylhexylester.

Durch Kombination von Verbindungen der Formel I mit Neo Heliopan® AP und einem UV-B-Filter, z.B. Ethylhexylmethoxycinnamat oder UV-B-Filtergemischen sowie beschichteten oder unbeschichteten feindispersen Metalloxiden wie z.B. Zinkoxid, Titandioxid wird eine UV-Breitband-Schutzleistung mit einer kritischen Wellenlänge $\lambda_{crit.}$> 380

nm erzielt *(vgl. hierzu Diffey in Int. J. Cosm. Science 16, 47 (1994))*.

Weiterhin lassen sich die erfindungsgemäßen Indanylidenverbindungen allein oder mit anderen UV-Absorbern, die für den Schutz technischer Produkte eingesetzt werden, kombinieren.

Beispiele solcher UV-Absorber sind Verbindungen aus der Reihe der Benzotriazole, Benzophenone, Triazine, Zimtsäureester sowie Oxalanilide.

Die erfindungsgemäßen Indanylidenverbindungen sind kristallin und müssen in kosmetischen Zubereitungen ausreichend gelöst werden, um das Problem der Rekristallisation nach längerer Lagerungszeit zu vermeiden. Eine ausreichende Menge der üblicherweise in kosmetischen Zubereitungen eingesetzten Ölkomponenten, flüssiger öllöslicher UV-Absorber oder Alkohole, z. B. Ethanol, Isopropanol oder 1-Butanol, ist zur Vermeidung der Rekristallisation erforderlich. Besonders bevorzugt ist der Einsatz folgender Ölkomponenten und/oder UV-Absorber zur Erzielung einer ausreichenden Löslichkeit von Verbindungen der erfindungsgemäßen Indanylidenverbindungen:

Ethylhexylmethoxycinnamat, Isoamyl-methoxycinnamat, Octocrylen, Ethylhexylsalicylat, Homosalat, Menthylanthranilat, Padimate O, Diisopropyladipat, $C_{12-15}$-Alkylbenzoat (Witconol TN), Butylenglykol-dicaprylat/-dicaprat (Miglyol 8810), Cocoglyceride (Myritol 331), Capryl/capr.-triglyceride (Miglyol 812), Cetearyl-isononanat (Cetiol SN), PVP/Hexadecen-Copolymer (Unimer U151), Adipinsäure-/Diethylenglykol/Isononansäure-Copolymer (Lexorez 100), Propylenglykoldicaprylat/ Dicaprat (Myritol PC), Hexyllaurat (Cetiol A), Dicaprylether (Cetiol OE), Diethylhexyl-naphthalat (Hallbrite® TQ), Butyloctylsalicylat (Hallbrite® BHB), Dibutyladipat (Cetiol B), Triethylcitrat (Hydagen CAT), Propylenglykol-dibenzoat (Finsolv PG 22), Tributylcitrat, Dioctylmalat (Ceraphyl 45), Dipropylenglykoldibenzoat (Benzoflex 245), Acetyl-tributylcitrat (Citroflex A-4), Acetyl-triethylcitrat (Citroflex A-2). Die Aufzählung der genannten Öle, die im Sinne der vorliegenden Erfindung eingesetzt werden können, soll selbstverständlich nicht limitierend sein.

Die Einsatzmenge aller Ölkomponenten in kosmetischen Emulsionen mit Verbindungen der Formel I ist 0,5 bis 30 Gew.-%, bevorzugt 2 bis 15 Gew.-%. Alle genannten Ölkomponenten bzw. flüssigen öllöslichen UV-Filter sind ausgezeichnete Lösungsmittel für alle kristallinen öllöslichen UV-Absorber.

Sehr nachteilig ist, wenn UV-Absorber auf Kleidungsstücken nicht mehr auswaschbare Flecken hinterlassen. Insbesondere ist von dem UV-A-Absorber tert.-Butylmethoxydibenzoylmethan bekannt, dass er nicht mehr auswaschbare Flecken auf Textilien erzeugt. Diesen Nachteil haben die erfindungsgemäßen Indanylidenverbindungen nicht, da eine Fleckenbildung auf Textilien sehr gut auswaschbar ist.

Sonnenschutzprodukte sollen wasserfest sein, damit ein ausreichender UV-Schutz für den Anwender, insbesondere Kinder, beim Schwimmen oder Baden gewährleistet ist. Verbindungen der erfindungsgemäßen Indanylidenverbindungen erfüllen diese Anforderungen in besonderem Maße. In einer O/W-Emulsion mit 3 Gew.-% einer Verbindung der erfindungsgemäßen Indanylidenverbindungen wurden 97 % Substantivität des UV-Absorbers nach dem Waschen gemessen und in einer W/O-Emulsion 95%. Weiterhin kann die Wasserfestigkeit von Sonnenschutzprodukten mit wasserlöslichen, einfach oder mehrfach sulfonierten UV-Filtem wie z.B. Neo Heliopan® AP, Mexoryl® SX, Benzophenon-4, Neo Heliopan® Hydro und den auf Seiten 6-9 aufgeführten öllöslichen UV-Absorbern durch Kombination mit Verbindungen der Formel I signifikant erhöht werden.

Es kann ferner von erheblichen Vorteil sein, die erfindungsgemäß genannten UV-Absorber mit chelatisierenden Substanzen, wie sie z.B. in EP-A 496 434, EP-A 313 305 und WO-94/04128 aufgeführt sind, oder mit Polyasparaginsäure und Ethylendiamintetramethyl-phosphonsäuresalzen zu kombinieren.

Kosmetische und dermatologische Formulierungen im Sinne der Erfindung enthalten einen oder mehrere übliche UV-A-, UV-B- und/oder Breitbandfilter als Einzelsubstanzen oder in beliebigen Gemischen untereinander, in der Lipidphase und/oder in der wässrigen Phase. Sie sind in jeglicher Hinsicht befriedigende Produkte, welche sich erstaunlicherweise durch eine hohe UV-A-Schutzleistung bzw. hohen Sonnenschutzfaktor auszeichnen.

Gegenstand der Erfindung ist ferner die Verwendung der erfindungsgemäßen UV-Absorber in Kombination mit herkömmlichen UV-Absorbern zur Verstärkung des Schutzes vor schädigender UV-Strahlung über das Maß des Schutzes, der bei Einsatz gleicher Mengen herkömmlicher oder der erfindungsgemäßen UV-Filter allein erzielt wird (synergistischer Effekt).

Die Gesamtmenge an UV-Filtersubstanzen (UV-A-, UV-B- und/ oder Breitbandfilter) in den fertigen kosmetischen oder dermatologischen Zubereitungen, sei es als Einzelsubstanz oder in beliebigen Gemischen untereinander, wird vorteilhaft aus dem Bereich 0,1 bis 30 Gew.-%, bevorzugt 0,1 bis 10,0 Gew.-%, insbesondere 0,5 bis 5,0 Gew.-% gewählt, bezogen auf das Gesamtgewicht der Zubereitungen.

Erfindungsgemäße kosmetische und dermatologische Zubereitungen enthalten ferner vorteilhaft, wenngleich nicht zwingend, anorganische Pigmente auf Basis von feindispersen Metalloxiden und/oder anderen in Wasser schwerlöslichen oder unlöslichen Metallverbindungen, insbesondere der Oxide des Titans ($TiO_2$) Zinks (ZnO), Eisens (z.B. $Fe_2O_3$), Zirkoniums ($ZrO_2$), Siliciums ($SiO_2$) Mangans (z.B. MnO), Aluminiums ($Al_2O_3$), Cers (z.B. $Ce_2O_3$), Mischoxiden der entsprechenden Metalle sowie Abmischungen aus solchen Oxiden. Diese Pigmente sind röntgenamorph oder nichtröntgenamorph. Besonders bevorzugt handelt es sich um Pigmente auf der Basis von $TiO_2$.

Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente durch Flammenreaktion erhältlich, beispielsweise dadurch, dass ein Metall- oder Halbmetallhalogenid mit Wasserstoff und Luft (oder reinem Sauerstoff) in einer Flamme umgesetzt wird.

In kosmetischen, dermatologischen oder pharmazeutischen Formulierungen werden röntgenamorphe Oxidpigmente als Verdickungs- und Thixotropierungsmittel, Fließhilfsmittel zur Emulsions- und Dispersionsstabilisierung und als Trägersubstanz (beispielsweise zur Volumenerhöhung von feinteiligen Pulvern oder Pudern) eingesetzt. Bekannte und in der kosmetischen oder dermatologischen Galenik oftmals verwendete röntgenamorphe Oxidpigmente sind beispielsweise hochreines Silicium- oxid. Bevorzugt werden hochreine, röntgenamorphe Siliciumdioxid-pigmente mit einer Partikelgröße im Bereich von 5 bis 40 nm und einer aktiven Oberfläche (BET) im Bereich von 50 bis 400 $m^2/g$, bevorzugt 150 bis 300 $m^2/g$, wobei die Partikel als kugelförmige Teilchen mit sehr einheitlicher Abmessung anzusehen sind. Makroskopisch sind die Siliciumdioxidpigmente als lockere, weiße Pulver erkenntlich. Siliciumdioxidpigmente werden im Handel unter dem Namen Aerosil® (CAS -Nr. 7631-85-9) oder Carb-O-Sil vertrieben

Vorteilhafte Aerosil® -Typen sind beispielsweise Aerosil® 0X50, Aerosil® 130, Aerosil® 150, Aerosil® 200, Aerosil® 300, Aerosil® 380, Aerosil® MQX 80, Aerosil® MOX 170, Aerosil® COK 84, Aerosil® R 202, Aerosil® R 805, Aerosil® R 812, Aerosil® R 972, Aerosil® R 974, Aerosil® R976.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% röntgenamorphe Oxidpigmente.

Die nicht-röntgenamorphen anorganischen Pigmente liegen erfindungsgemäß vorteilhaft in hydrophober Form vor, d.h., dass sie oberflächlich wasserabweisend behandelt sind. Diese Oberflächenbehandlung kann darin bestehen, dass die Pigmente nach an sich bekannten Verfahren mit einer dünnen hydrophoben Schicht versehen werden. Eines solcher Verfahren besteht beispielsweise darin, dass die hydrophobe Oberflächenschicht nach einer Reaktion gemäß

$$n\ TiO_2 + m\ (RO)_3Si\text{-}R` \rightarrow n\ TiO_2\ \text{(oberfl.)}$$

erzeugt wird. n und m sind dabei nach Belieben einzusetzende stöchiometrische Parameter, R und R' die gewünschten organischen Reste. Beispielsweise in Analogie zu DE-A 33 14 742 dargestellte hydrophobisierte Pigmente sind von Vorteil.

Beispielsweise seien $TiO_2$-Pigment genannt, wie sie unter der Handelsbezeichnung T805 von der Firma Degussa vertrieben werden. Bevorzugt sind auch $TiO_2/Fe_2O_3$-Mischoxide, wie sie beispielsweise unter der Handelsbezeichnung T817 ebenfalls von der Firma Degussa angeboten werden.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 bis 30 Gew.-%, bevorzugt 0,1 bis 10,0, insbesondere 0.5 bis 6.0 Gew.-% gewählt, bezogen auf das Gesamtgewicht der Zubereitungen.

Die erfindungsgemäßen kosmetischen und/oder dermatologischen Formulierungen können wie üblich zusammengesetzt sein und dem kosmetischen und/oder dermatologischen Sonnenschutz, ferner zur Behandlung, der Pflege und der Reinigung der Haut und/oder der Haare und als Schminkprodukt in der dekorativen Kosmetik dienen. Entsprechend können die erfindungsgemäßen Zubereitungen, je nach ihrem Aufbau, beispielsweise verwendet werden als Hautschutzcreme, Reinigungsmilch, Sonnenschutzlotion, Nährcreme, Tages- oder Nachtcreme usw. Es ist gegebenenfalls möglich und vorteilhaft, die erfindungsgemäßen Zubereitungen als Grundlage für pharmazeutische Formulierungen zu verwenden. Bevorzugt sind insbesondere solche kosmetischen und dermatologischen Zubereitungen, die in der Form eines Hautpflege- bzw. Schminkproduktes vorliegen. Typische Ausgestaltungen sind Cremes, Gele, Lotionen, alkoholische und wäßrig/alkoholische Lösungen, Emulsionen oder Stiftpräparate. Diese Mittel können ferner als weitere Hilfs- und Zusatzstoffe milde Tenside, Co-Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deowirkstoffe, Antischuppenmittel, Filmbildner, Quellmittel, Hydrotrope, Konservierungsmittel, Insektenrepellentien, Bräunungsmittel, künstliche Selbstbräunungsmittel (z.B. Dihydroxyaceton), Solubilisatoren, Parfümöle, Farbstoffe, keimhemmende Mittel und dergleichen enthalten.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines kosmetischen Mittels zum Schutz von Haut und Haaren vorliegen. Vorteilhaft können diese zusätzlich zu erfindungsgemäß verwendeten UV-A, UV-B- und/oder Breitbandfiltern mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatolo-gischen Formulierung wie Alkohole, Polyole, Polymere, Schaumstabilisatoren, Elektrolyte, organische Lösungsmittel oder Silikonderivate. Als nicht ionische Emulgatoren bzw. Dispergatoren kommen in Frage die Gruppe, die gebildet wird von Polyglyceryl-2-dipolyhydroxystearate (Dehymuls® PGPH), Polyglyceryl-3-diiso-stearate (Lameform® TGI), Polyglyceryl-4-isostearate (Isolan® GI 34), Polyglyceryl-3-oleate, Diisostearyl-Polyglyceryl-3-diisostearate (Isolan® PDI), Polyglyceryl-3-methylglucose distearate (Tego Carey® 450), Polyglyceryl-3-beeswax (Cera Bellina® ), Polyglyceryl-4-caprate (Polyglycerol caprate T2010/90), Polyglyceryl-3-cetylether (Chimexane® NL), Polyglyceryl-3-distearate (Cremophor® GS 32), Polyglyceryl-2-stearate (Hostacerin® DGMS) und Polyglyceryl-polyricineoleate (Admul® WOL 1403) sowie deren Gemischen.

Die jeweils einzusetzenden Mengen an kosmetischen oder dermatologischen Hilfsund Trägerstoffen und Parfüm können in Abhängigkeit von der Art des jeweiligen Produktes vom Fachmann durch einfaches Ausprobieren leicht ermittelt werden.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyro-phenon, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse;
- Öle, wie Triglyceride der Caprin- oder der Caprylsäure, ferner natürlicher Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxan, Diethylpolysiloxan, Diphenylpolysiloxan sowie Mischformen daraus.

Die Ölphasen der Emulsionen, Oleogele bzw. Hydrodispersionen oder Lipodispersionen im Sinne der vorliegenden Erfindung werden vorteilhaft gewählt aus der Gruppe der Ester aus gesättigten und/oder ungesättigten, verzweigten und/oder unverzweig-ten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, -palmitat, - stearat, -oleat, n-Butylstearat, n-Hexyllaurat, n-Decyloleat, Isooctylstearat, Isononylstearat, Isononylisononanat, 2-Ethyl-hexylpalmitat, Ethylhexyllaurat, 2-Hexyldecylstearat, 2-Octyldodecylpalmitat, Oleyloleat, Oleylerucat, Erucyloleat sowie synthetische, halbsynthetische und natürliche Gemische solcher Ester, zB. Jojobaöl.

Ferner kann die Ölphase vorteilhaft gewählt werden aus der Gruppe der verzweigten und unverzweigten Kohlenwasserstoffe und -wachse, der Siliconöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder Siliconölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole (Niederalkyl), sowie deren Ether, vorzugsweise Ethanol, Isopropanol, Propylenglykol, Glycerin, Ethylenglykol-monoethyl- oder monobutylether, Propylenglykolmonomethyl, -monoethyl- oder monobutyl-ether, Diethylenglykolmonomethyl- oder -monoethylether und analoge Produkte, ferner Alkohole (Niederalkyl), z.B. Ethanol, 1,2-Propandiol, Glycerin sowie insbe-sondere ein oder mehrere Verdickungsmittel, welches oder welche vorteilhaft ge-wählt werden können aus der Gruppe Siliciumdioxid, Aluminiumsilikate, Poly -saccharide bzw. deren Derivate, z.B. Hyaluronsäure, Xanthangummi, Hydroxy-propylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombina-tion.

Eine umfassende Beschreibung der in kosmetischen Mitteln eingesetzten Roh- und Wirkstoffe ist in DE-A 199 19 630 dargestellt.

Es war nicht vorauszusehen, dass die erfindungsgemäßen Indanylidenverbindungen im Vergleich zu den aus der EP-A 823 418 bekannten Verbindungen eine herausragende Auswahl darstellen.

**Beispiele:**

**Photostabilität**

Im folgenden werden beispielhaft Vergleichsmessungen zwischen den Verbindungen der Kategorie A und den Verbindungen der Kategorie B sowie die Kombination mit anderen marktüblichen UV-Filtern wie OMC (= Octylmethoxycinnamat) oder BMDM (= tert.-Butyl-methoxy-dibenzoylmethan) angeführt. Die Substanzen der Kategorie B zeigen den Fortschritt gegenüber den Substanzen der Kategorie A. Die Bestrahlung wurde in einem Suntester der Firma Heraeus mit einer Bestrahlungsstärke von 765 $W/m^2$ (bez. auf Globalsensor) durchgeführt. Die Werte geben die Konzentrationsabnahme der UV-Filter in Prozent nach Bestrahlung (Dosis in $J/cm^2$) wieder.

Formulierung nach Rezepturbeispiel 1

| Verbindung | A1 | A2 | B1 | B2 | B3 |
|---|---|---|---|---|---|
| 72 J/cm² | 10% | 1% | 2% | 1% | 1% |
| 144 J/cm² | 13% | 6% | 3% | 2% | 2% |

Tab.1

Formulierung nach Rezepturbeispiel 12

| Kombination | A1 | OMC | A2 | OMC | B1 | OMC | B2 | OMC | B3 | OMC |
|---|---|---|---|---|---|---|---|---|---|---|
| 72 J/cm² | 22% | 27% | 14% | 7% | 2% | 12% | 6% | 11% | 6% | 11% |
| 144 J/cm² | 37% | 35% | 26% | 12% | 3% | 19% | 6% | 13% | 6% | 13% |

Tab.2

Formulierung nach Rezeptur

| | *Vergleich ohne A oder B* | | *Beispiel 12* | | *Beispiel 4 ohne OMC* | | *Beispiel 4* | | |
|---|---|---|---|---|---|---|---|---|---|
| Kombination | OMC | BMDM | B3 | OMC | B3 | BMDM | B3 | OMC | BMDM |
| 72 J/cm² | 48% | 68% | 6% | 11% | 2% | 5% | 3% | 17% | 35% |
| 144 J/cm² | 59% | 85% | 6% | 13% | 3% | 13% | 5% | 26% | 54% |

Tab.3

**Verbindungen in Tabelle 1-3:**

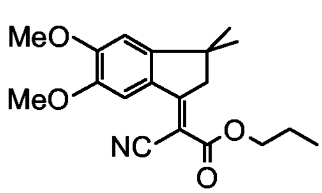

A1

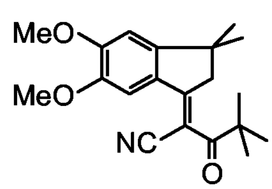

B1

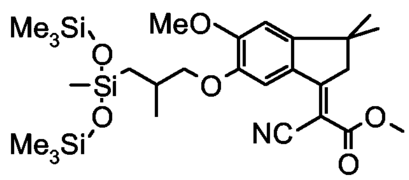

A2

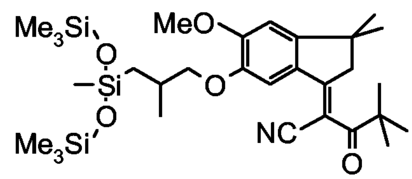

B2

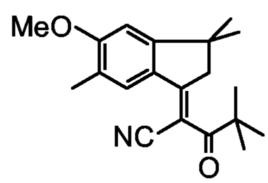

B3

**Beispiel 1**

2-(5,6-Dimethoxy-3,3-dimethyl-1-indanyliden)-4,4-dimethyl-3-oxo-pentansäurenitril

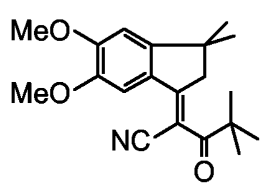

44 g (0,2 mol) 5,6-Dimethoxy-3,3-dimethyl-1-indanon, 25 g (0,2 mol) Pivaloylacetonitril, 32 g Propionsäure und 17 g Ammoniumacetat werden in 80 g Xylol gemischt und auf 120°C 7 Stunden erhitzt. Nach Abkühlen auf Raumtemperatur und Waschen der organischen Phase wird das Xylol abdestilliert und das verbleibende Rohprodukt in Methanol umkristallisiert. Ausbeute: 50 % der Theorie; $E^{1/1}$ 730 ($\lambda_{max}$ 373 nm).

**Beispiel 2**

2-(5-Methoxy-3,3,4,6-tetramethyl-1-indanyliden)-4,4-dimethyl-3-oxo-pentansäurenitril

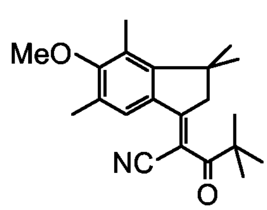

Es wurde analog zu Beispiel 1 ausgehend von 5-Methoxy-3,3,4,6-tetramethyl-1-indanon vorgegangen. Ausbeute: 50 % der Theorie; $E^{1/1}$ 588 ($\lambda_{max}$ 340 nm).

**Beispiel 3**

2-(3,3,5,6-tetramethyl-1-indanyliden)-4,4-dimethyl-3-oxo-pentansäurenitril

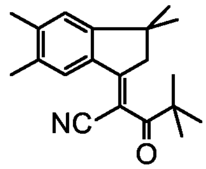

Es wurde analog zu Beispiel 1 ausgehend von 3,3,5,6-tetramethyl-1-indanon vorgegangen. Ausbeute: 55 % der Theorie; $E^{1/1}$ 630 ($\lambda_{max}$ 342 nm).

**Beispiel 4**

2-(3,3,6-trimethyl-1-indanyliden)-4,4-dimethyl-3-oxo-pentansäurenitril

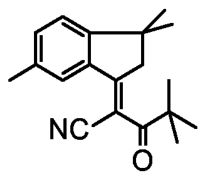

Es wurde analog zu Beispiel 1 ausgehend von 3,3,6-trimethyl-1-indanon vorgegangen. Ausbeute: 45 % der Theorie; $E^{1/1}$ 528/505 ($\lambda_{max}$ 335/316 nm).

**Beispiel 5**

2-(5,6-Ethylendioxo-3,3-dimethyl-1-indanyliden)-4,4-dimethyl-3-oxo-pentansäurenitril

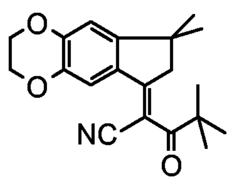

Es wurde analog zu Beispiel 1 ausgehend von 5,6-Ethylendioxo-3,3-dimethyl-1-indanon vorgegangen. Ausbeute: 55 % der Theorie; $E^{1/1}$ 640 ($\lambda_{max}$ 369 nm).

**Beispiel 6**

2-(5-Methoxy-3,3,6-trimethyl-1-indanyliden)-4,4-dimethyl-3-oxo-pentansäurenitril

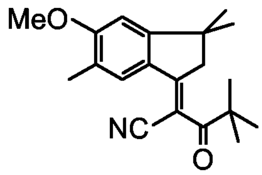

Es wurde analog zu Beispiel 1 ausgehend von 5-Methoxy-3,3,6-trimethyl-1-indanon vorgegangen. Ausbeute: 60 % der Theorie; $E^{1/1}$ 850 ($\lambda_{max}$ 359 nm).

**Beispiel 7**

2-[(5-Methoxy-3,3-dimethyl-(2-methyl-3-(1,3,3,3-tetramethyl-1 -(trimethyl-silyloxy)-disiloxanyl)-propyl )-indanyliden)]-4,4-dimethyl-3-oxo-pentan-säurenitril

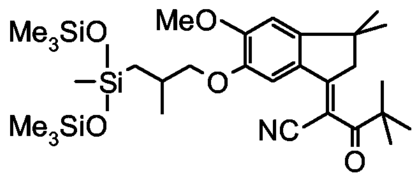

a) 2-(5-Methoxy-3,3-dimethyl-6-hydroxy-1-indanyliden)-4,4-dimethyl-3-oxopentan-säurenitril

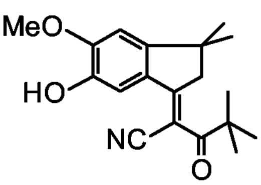

5-Methoxy-3,3-dimethyl-6-hydroxy-1-indanon wird nach dem Beispiel 1 umgesetzt. Ausbeute: 50% d.Th.

b) 2-(5-Methoxy-3,3-dimethyl-6-(2-methyl-propenyloxy)-1-indanyliden)-4,4-dimethyl-3-oxo-pentansäurenitril

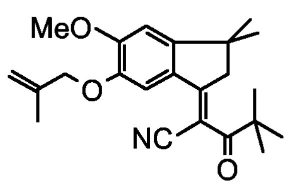

136 g (0,43 mol) der Verbindung unter a) werden zusammen mit 95 g Kaliumcarbonat in 470 g N-Methylpyrrolidinon gegeben, auf 70°C erhitzt und innerhalb von 30 min mit 42 g (0,46 mol) Methallylchlorid versetzt. Man erhitzt noch 3 h bei 70°C, kühlt ab auf Raumtemperatur und extrahiert das Produkt mit Essigester. Ausbeute: 45% d.Th.

90g (130 mmol) der Verbindung unter b), 29g (130 mmol) 1,1,1,3,5,5,5-Heptamethyltrisiloxan werden in Gegenwart katalytischer Mengen Divinyl-tetramethyl-Platin-Komplex in 90g Toluol und Stickstoffatmosphäre 20h auf 80°C gehalten. Nach Abdestillieren des Lösungsmittels wird der Rückstand über ein Kugelrohr destilliert. Man erhält 50g (70% d. Th.) des gewünschten Produktes als gelbes Öl; $E^{1/1}$ 400 ($\lambda_{max}$ 373 nm).

**Beispiel 8**

2-(6-Acetoxy-3,3-dimethyl-5-methoxy-1-indanyliden)-4,4-dimethyl-3-oxo-pentansäurenitril

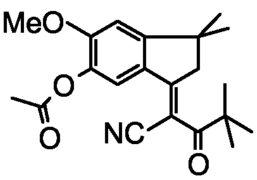

54 g (0,17 mol) der Verbindung unter a) werden mit 13 g (0,17 mol) Acetylchlorid in N-Methylpyrrolidinon bei 40°C innerhalb 5h umgesetzt. Ausbeute: 98 % d.Th.
$E^{1/1}$ 420/280 ($\lambda_{max}$ 355/302 nm).

**Beispiel 9**

2-(3,3-Dimethyl-5-tert.-butyl-1-indanyliden)-3-(1'-methylcyclohexyl)-3-oxopropionsäurenitril

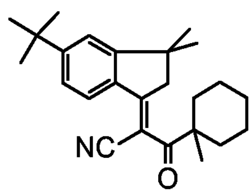

3,3-Dimethyl-5-tert.-butyl-1-indanon werden mit 3-(1'-Methylcyclohexyl)-3-oxopropionsäurenitril analog zu Beispiel 1 umgesetzt.
Ausbeute: 40 % d.Th.
$E^{1/1}$ 580 ($\lambda_{max}$ 355 nm).

**Beispiel 10**

2-(3,3,5-Trimethyl-1-indanyliden)-3-phenyl-3-oxo-propionsäurenitril

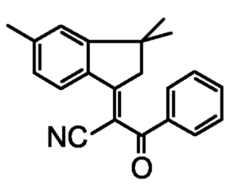

3,3,5-Trimethyl-1-indanon werden mit Benzoylacetonitril analog zu Beispiel 1 umgesetzt.
Ausbeute: 50 % d.Th.
$E^{1/1}$ 600 ($\lambda_{max}$ 350 nm).

| Rezepturbeispiel 1 | | | |
|---|---|---|---|
| Sonnenschutz Soft Creme (O/W), in-vitro SPF 3, wasserfest | | | |
| Teil | Rohstoffe | INCI Bezeichnung | % (Gew.) |
| A | Crodafos MCA | Cetyl Phosphate | 1.50 |
| | Cutina MD | Glyceryl Stearate | 2.00 |
| | Copherol 1250 | Tocopherylacetate | 0.50 |
| | Lanette 16 | Cetyl Alcohol | 1.00 |
| | Tegosoft TN | C 12-15 Alkyl Benzoate | 24.00 |
| | Prisorine 3505 | Isostearic Acid | 1.00 |
| | UV-Absorber gemäß Formel I | | 3.00 |
| | | | |
| B | Wasser, dest. | Water (Aqua) | 59.60 |
| | EDETA B fl. | Tetrasodium EDTA | 0.20 |
| | Glycerin, 99% | Glycerin | 3.00 |
| | Phenoxyethanol | Phenoxyethanol | 0.70 |
| | Solbrol M | Methylparaben | 0.20 |
| | Solbrol P | Propylparaben | 0.10 |
| | Carbopol ETD 2050 | Carbomer | 0.20 |
| | | | |
| C | Natronlauge, 10 % aq. | Sodium Hydroxide | 2.70 |
| | | | |
| D | Parfümöl | Parfum (Fragrance) | 0.30 |

**Herstellungsverfahren**

Teil A: Auf ca. 85°C erhitzen.
Teil B: Rohstoffe ohne Carbopol einwiegen. Carbopol mit Ultra Turrax eindispergieren. Auf ca. 85°C erhitzen. B zu A geben.
Teil C: Sofort zu A/B geben und anschließend heiß homogenisieren (Ultra Turrax).
Unter Rühren abkühlen lassen.
Teil D: Zugeben und verrühren.

| | |
|---|---|
| in-vitro SPF (Labsphere Ultraviolet Transmittance Analyzer) | 3 |
| Boots Star Rating | 4 |
| Broad Spectrum Rating (Diffey) | 4 |
| Kritische Wellenlänge (90% Absorption bei λ in nm) | 385 |
| UV-Filter Substantivität nach Wässerung | 97 % |

| Rezepturbeispiel 2 | | | |
|---|---|---|---|
| Sonnenschutzlotion (O/W), in-vitro SPF 20 | | | |
| Teil | Rohstoffe | INCI Bezeichnung | % (Gew.) |
| A | Crodafos MCA | Cetyl Phosphate | 1.50 |
| | Cutina MD | Glyceryl Stearate | 2.00 |
| | Copherol 1250 | Tocopherylacetate | 0.50 |
| | Lanette 16 | Cetyl Alcohol | 1.00 |
| | Tegosoft TN | C 12-15 Alkyl Benzoate | 10.60 |
| | Prisorine 3505 | Isostearic Acid | 1.00 |
| | UV-Absorber gemäß Formel I | | 2.00 |
| | Neo Heliopan® AV | Ethyl Hexyl Methoxycinnamate | 5.00 |
| | | | |
| B | Wasser, dest. | Water (Aqua) | 55.07 |
| | EDETA B fl. | Tetrasodium EDTA | 0.20 |
| | Glycerin, 99% | Glycerin | 3.00 |
| | Phenoxyethanol | Phenoxyethanol | 0.70 |
| | Solbrol M | Methylparaben | 0.20 |
| | Solbrol P | Propylparaben | 0.10 |
| | Carbopol ETD 2050 | Carbomer | 0.20 |
| | | | |
| C | Natronlauge, 10 % aq. | Sodium Hydroxide | 3.30 |
| | Neo Heliopan® Hydro, 15% ige Lösung neutralisiert mit NaOH | Phenylbenzimidazole Sulfonic Acid | 13.33 |
| | | | |
| D | Parfümöl | Parfum (Fragrance) | 0.30 |

**Herstellungsverfahren**

Teil A: Auf ca. 85°C erhitzen.
Teil B: Rohstoffe ohne Carbopol einwiegen. Carbopol mit Ultra Turrax eindispergieren. Auf ca. 85°C erhitzen. B zu A geben.
Teil C: Sofort zu A/B geben und anschließend heiß homogenisieren (Ultra Turrax). Unter Rühren abkühlen lassen.
Teil D: Zugeben und verrühren.

| | |
|---|---|
| in-vitro SPF (Labsphere Ultraviolett Transmittance Analyzer) | 20 |
| Boots Star Rating | 2 |
| Broad Spectrum Rating (Diffey) | 4 |
| Kritische Wellenlänge (90% Absorption bei λ in nm) | 378 |

| **Rezepturbeispiel 3** | | | |
|---|---|---|---|
| Sonnenschutzmilch (O/W), in-vitro SPF 6 | | | |
| Teil | Rohstoffe | INCI Bezeichnung | % (Gew.) |
| A | Tegin M | Glyceryl Stearate | 2.50 |
| | Tagat S | PEG-30 Glyceryl Stearate | 1.95 |
| | Lanette O | Cetearyl Alcohol | 2.20 |
| | Copherol 1250 | Tocopherylacetat | 0.50 |
| | Miglyol 8810 | Butylene Glycol Dicaprylate/ Caprate | 12.00 |
| | Tegosoft TN | C 12 - C 15 Alkylbenzoate | 8.00 |
| | Phenonip | Phenoxyethanol (and) Methylparaben (and) Butylparaben (and) Ethyl-paraben (and) Propylparaben | 0.15 |
| | UV-Absorber gemäß Formel I | | 5.00 |
| | | | |
| B | Wasser, dest. | Water (Aqua) | 43.90 |
| | EDETA BD | Disodium EDETA | 0.10 |
| | 1,2-Propylenglykol | Propylene Glycol | 2.00 |
| | Phenonip | Phenoxyethanol (and) Methylparaben (and) Butylparaben (and) Ethylparaben (and) Propylparaben | 0.30 |
| | | | |
| C | Wasser, dest. | Water (Aqua) | 19.00 |
| | Carbopol 2050 | Carbomer | 0.40 |
| | NaOH, 10%ig | Sodium Hydroxide | 1.70 |
| | | | |
| D | Parfümöl | Parfum (Fragrance) | 0.30 |

**Herstellungsverfahren**

Teil A: Auf 80-85°C erhitzen.
Teil B: Auf 80-85°C erhitzen, unter Rühren Teil B zu Teil A geben.
Teil C: Carbopol in das Wasser eindispergieren und unter Rühren mit NaOH neutralisieren. Teil C bei ca. 60°C unter Rühren zugeben. Auf RT abkühlen lassen.
Teil D: Zugeben und verrühren.

| | |
|---|---|
| in-vitro SPF (Labsphere Ultraviolet Transmittance Analyzer) | 6 |
| Boots Star Rating | 4 |
| Broad Spectrum Rating (Diffey) | 4 |
| Kritische Wellenlänge (90% Absorption bei λ in nm) | 385 |

| **Rezepturbeispiel 4** | | | |
|---|---|---|---|
| Sonnenschutzlotion (O/W), in-vitro SPF 21 | | | |
| Teil | Rohstoffe | INCI Bezeichnung | % (Gew.) |
| A | Tegin M | Glyceryl Stearate | 2.50 |
| | Tagat S | PEG-30 Glyceryl Stearate | 1.95 |
| | Lanette O | Cetearyl Alcohol | 2.20 |
| | Copherol 1250 | Tocopherylacetat | 0.50 |
| | Miglyol 8810 | Butylene Glycol Dicaprylate / Caprate | 12.00 |
| | Tegosoft TN | C12 - C15 Alkylbenzoate | 8.00 |
| | Phenonip | Phenoxyethanol (and) Methylparaben (and) Butylparaben (and) Ethylparaben (and) Propylparaben | 0.20 |
| | UV-Absorber gemäß Formel I | | 2.00 |
| | Neo Heliopan® AV | Ethylhexyl Methoxycinnamate | 5.00 |
| | Neo Heliopan® 357 | Butyl Methoxydibenzoylmethane | 1.00 |
| B | Wasser, dest. | Water (Aqua) | 39.35 |
| | EDETA BD | Disodium EDETA | 0.10 |
| | 1,2-Propylenglykol | Propylene Glycol | 2.00 |
| | Phenonip | Phenoxyethanol (and) Methylparaben (and) Butylparaben (and) Ethylparaben (and) Propylparaben | 0.30 |
| | Vitamin C | Ascorbic Acid | 0.10 |
| | | | |
| C | Wasser, dest. | Water (Aqua) | 20.00 |
| | Carbopol 2050 | Carbomer | 0.40 |
| | NaOH, 10%ig | Sodium Hydroxide | 1.70 |
| | | | |
| D | Parfümöl | Parfum (Fragrance) | 0.30 |

**Herstellungsverfahren**

Teil A: Auf 80-85°C erhitzen.
Teil B: Auf 80-85°C erhitzen, unter Rühren Teil B zu Teil A geben.
Teil C: Carbopol in das Wasser eindispergieren und unter Rühren mit NaOH neutralisieren. Teil C bei ca. 60°C unter Rühren zugeben. Auf RT abkühlen lassen.
Teil D: Zugeben und verrühren.

| | |
|---|---|
| in-vitro SPF (Labsphere Ultraviolet Transmittance Analyzer) | 21 |
| Boots Star Rating | 3 |
| Broad Spectrum Rating (Diffey) | 4 |
| Kritische Wellenlänge (90% Absorption bei λ in nm) | 379 |

| **Rezepturbeispiel 5** | | | |
|---|---|---|---|
| Sonnenschutzlotion (O/W), in-vitro SPF 11 | | | |
| Teil | Rohstoffe | INCI Bezeichnung | % (Gew.) |
| A | Eumulgin VL 75 | Lauryl Glucoside (and) Polyglyceryl-2-Dipolyhydroxystearate (and) Glycerin | 3.00 |
| | Tegosoft TN | C12-25 Alkyl Benzoate | 20.00 |
| | Copherol 1250 | Tocopherylacetat | 0.50 |
| | UV-Absorber gemäß Formel I | | 3.00 |
| | Parfümöl | Parfum (Fragrance) | 0.20 |
| | Neo Heliopan® 303 | Octocrylene | 5.00 |
| | Carbopol 2984 | Carbomer | 0.35 |
| | Pemulen TR-1 | Acrylates/C10-30 Alkylacrylate Crosspolymer | 0.15 |
| | | | |
| B | Wasser, dest. | Water (Aqua) | 60.50 |
| | EDETA BD | Disodium EDTA | 0.10 |
| | Glycerin, 99% | Glycerin | 5.00 |
| | Phenoxyethanol | Phenoxyethanol | 0.70 |
| | Solbrol M | Methylparaben | 0.20 |
| | Solbrol P | Propylparaben | 0.10 |
| | | | |
| C | NaOH, 10%ig | Sodium Hydroxide | 1.20 |

**Herstellungsverfahren**

Teil A: UV-Absorber gemäß Formel 1 in den Ölen bzw flüssigen UV-Filtem lösen (Erwärmen bis ca. 70°C). Abkühlen lassen auf ca. 30°C, restliche Bestandteile außer Carbopol und Pemulen zufügen und bei Raumtemperatur mischen (ca. 5 Minuten rühren). Carbopol und Pemulen einrühren.

Teil B: Solbrole unter Erwärmen in Phenoxyethanol lösen. Mit Wasser und Glycerin mischen, unter Rühren zu Teil A geben. Ca. 60 Minuten rühren.

Teil C: Zu A/B geben, mit dem Ultra Turrax homogenisieren.

| | |
|---|---|
| in-vitro SPF (Labsphere Ultraviolet Transmittance Analyzer) | 11 |
| Boots Star Rating | 4 |
| Broad Spectrum Rating (Diffey) | 4 |
| Kritische Wellenlänge (90% Absorption bei λ in nm) | 382 |

| **Rezepturbeispiel 6** | | | |
|---|---|---|---|
| Sonnenschutzcreme (W/O) , in-vitro SPF 4, wasserfest | | | |
| Teil | Rohstoffe | INCI Bezeichnung | % (Gew.) |
| A | Dehymuls PGPH | Polyglyceryl-2 Dipolyhydroxystearate | 5.00 |

(fortgesetzt)

| Rezepturbeispiel 6 | | | |
|---|---|---|---|
| Sonnenschutzcreme (W/O) , in-vitro SPF 4, wasserfest | | | |
| Teil | Rohstoffe | INCI Bezeichnung | % (Gew.) |
| | Copherol 1250 | Tocopherylacetat | 0.50 |
| | Permulgin 3220 | Ozokerite | 0.50 |
| | Zinkstearat | Zinc Stearate | 0.50 |
| | Tegosoft TN | C12-15 Alkyl Benzoate | 25.00 |
| | UV-Absorber gemäß Formel I | | 5.00 |
| | | | |
| B | Wasser, dest. | Water (Aqua) | 57.90 |
| | EDETA BD | Disodium EDTA | 0.10 |
| | Glycerin, 99% | Glycerin | 4.00 |
| | Phenoxyethanol | Phenoxyethanol | 0.70 |
| | Solbrol M | Methylparaben | 0.20 |
| | Solbrol P | Propylparaben | 0.10 |
| | Magnesiumsulfat | Magnesium Sulfate | 0.50 |

<u>**Herstellungsverfahren**</u>

Teil A: Auf ca. 85°C erhitzen.
Teil B: Auf ca. 85°C erhitzen (ohne Zinkoxid; Zinkoxid mit dem Ultra Turrax eindispergieren).
B zu A geben.
Unter Rühren abkühlen lassen, anschließend homogenisieren.

| | |
|---|---|
| in-vitro SPF (Labsphere Ultraviolet Transmittance Analyzer) | 4 |
| Boots Star Rating | 4 |
| Broad Spectrum Rating (Diffey) | 4 |
| Kritische Wellenlänge (90% Absorption bei λ in nm) | 384 |
| UV-Filter Substantivität nach Wässerung | 95 % |

| Rezepturbeispiel 7 | | | |
|---|---|---|---|
| Sonnenschutz Softcreme (W/O) , in-vitro SPF 40 | | | |
| Teil | Rohstoffe | INCI Bezeichnung | % (Gew.) |
| A | Dehymuls PGPH | Polyglyceryl-2 Dipolyhydroxystearate | 5.00 |
| | Copherol 1250 | Tocopherylacetat | 0.50 |
| | Permulgin 3220 | Ozokerite | 0.50 |
| | Zinkstearat | Zinc Stearate | 0.50 |
| | Tegosoft TN | C12-15 Alkyl Benzoate | 10.00 |
| | UV-Absorber gemäß Formel I | | 2.00 |

(fortgesetzt)

| Rezepturbeispiel 7 | | | |
|---|---|---|---|
| Sonnenschutz Softcreme (W/O) , in-vitro SPF 40 | | | |
| Teil | Rohstoffe | INCI Bezeichnung | % (Gew.) |
| | Neo Heliopan® 303 | Octocrylene | 5.00 |
| | Neo Heliopan® MBC | 4-Methylbenzylidene Camphor | 3.00 |
| | Zinkoxid neutral H&R | Zinc Oxide | 5.00 |
| | | | |
| B | Wasser, dest. | Water (Aqua) | 62.90 |
| | EDETA BD | Disodium EDTA | 0.10 |
| | Glycerin, 99% | Glycerin | 4.00 |
| | Phenoxyethanol | Phenoxyethanol | 0.70 |
| | Solbrol M | Methylparaben | 0.20 |
| | Solbrol P | Propylparaben | 0.10 |
| | Magnesiumsulfat | Magnesium Sulfate | 0.50 |
| | | | |
| C | Parfümöl | Parfum (Fragrance) | 0.20 |

**Herstellungsverfahren**

Teil A: Auf ca. 85°C erhitzen.
Teil B: Auf ca. 85°C erhitzen (ohne Zinkoxid; Zinkoxid mit dem Ultra Turrax eindispergieren).
B zu A geben.
Unter Rühren abkühlen lassen.
Teil C: Zugeben und anschließend homogenisieren

| | |
|---|---|
| in-vitro SPF (Labsphere Ultraviolet Transmittance Analyzer) | 40 |
| Boots Star Rating | 3 |
| Broad Spectrum Rating (Diffey) | 4 |
| Kritische Wellenlänge (90% Absorption bei λ in nm) | 379 |

| Rezepturbeispiel 8 | | | |
|---|---|---|---|
| Sonnenschutzmilch (W/O) | | | |
| Teil | Rohstoffe | INCI Bezeichnung | % (Gew.) |
| A | Dehymuls PGPH | Polyglyceryl-2 Dipolyhydroxystearate | 3.00 |
| | Bienenwachs 8100 | Beeswax | 1.00 |
| | Monomuls 90-0-18 | Glyceryl Oleate | 1.00 |
| | Zinkstearat | Zinc stearate | 1.00 |
| | Cetiol SN | Cetearyl Isononanoate | 5.00 |
| | Cetiol OE | Dicaprylyl Ether | 5.00 |

(fortgesetzt)

| Rezepturbeispiel 8 | | | |
|---|---|---|---|
| Sonnenschutzmilch (W/O) | | | |
| Teil | Rohstoffe | INCI Bezeichnung | % (Gew.) |
| | Tegosoft TN | C12-15 Alkyl Benzoate | 4.00 |
| | Copherol 1250 | Tocopherylacetat | 0.50 |
| | Solbrol P | Propylparaben | 0.10 |
| | Neo Heliopan® OS | Ethylhexyl Salicylate | 5.00 |
| | Neo Heliopan® AV | Ethylhexyl Methoxycinnamate | 7.50 |
| | UV-Absorber gemäß Formel I | | 1.50 |
| | | | |
| B | Wasser, dest. | Water (Aqua) | 44.10 |
| | Trilon BD | Disodium EDTA | 0.10 |
| | Glycerin, 99% | Glycerin | 5.00 |
| | Solbrol M | Methylparaben | 0.20 |
| | Phenoxyethanol | Phenoxyethanol | 0.70 |
| | Neo Heliopan® AP 10%ige Lösung neutralisiert mit NaOH | Disodium Phenyl Dibenzimidazole Tetrasulfonate | 15.00 |
| | | | |
| C | Parfümöl | Parfum (Fragrance) | 0.30 |
| | Bisabolol | Bisabolol | 0.10 |

**Herstellungsverfahren**

Teil A: Auf ca. 85°C erhitzen.
Teil B: Auf ca. 85°C erhitzen. B zu A geben.Unter Rühren abkühlen lassen.
Teil C: Zugeben und anschließend homogenisieren.

| Rezepturbeispiel 9 | | | |
|---|---|---|---|
| Tagespflegecreme mit UV-Schutz | | | |
| Teil | Rohstoffe | INCI Bezeichnung | % (Gew.) |
| A | Emulgade PL 68/50 | Cetearyl Glycoside (and) Cetearyl Alcohol | 4.50 |
| | Cetiol PGL | Hexyldecanol (and) Hexyldecyl Laurate | 8.00 |
| | Myritol 331 | Cocoglycerides | 8.00 |
| | Copherol 1250 | Tocopheryl Acetat | 0.50 |
| | Neo Heliopan® E1000 | Isoamyl-p- Methoxycinnamate | 2.00 |
| | UV-Absorber gemäß Formel I | | 2.00 |
| | | | |
| B | Wasser, dest | Water (Aqua) | 45.40 |
| | Glycerin | Glycerin | 3.00 |

(fortgesetzt)

| **Rezepturbeispiel 9** | | | |
|---|---|---|---|
| Tagespflegecreme mit UV-Schutz | | | |
| Teil | Rohstoffe | INCI Bezeichnung | % (Gew.) |
| | Phenonip | Phenoxyethanol (and) Methylparaben (and) Butyparaben (and) Ethyparaben (and) Propylparaben | 0.50 |
| | | | |
| C | Wasser, dest | Water (Aqua) | 25.00 |
| | Carbopol ETD 2050 | Carbomer | 0.20 |
| | NaOH, 10%ig | Sodium Hydroxide | 0.60 |
| | | | |
| D | Parfümöl | Parfum ( Fragrance) | 0.30 |

**Herstellungsverfahren**

Teil A: Auf 80°C erhitzen.
Teil B: Auf 80°C erhitzen.Unter Rühren zu Teil A geben.
Teil C: Carbopol in Wasser dispergieren und mit Natronlauge neutralisieren. Bei ca. 55°C zu TeilA/Bgeben.
Teil D: Bei RT zugeben und homogenisieren.

| **Rezepturbeispiel 10** | | | |
|---|---|---|---|
| Sonnenschutzspray | | | |
| Teil | Rohstoffe | INCI Bezeichnung | % (Gew.) |
| A | Wasser, dem. | Water (Aqua) | 69.50 |
| | Glycerin, 99% | Glycerin | 4.00 |
| | 1,3-Butylenglykol | Butylene Glycol | 5.00 |
| | D-Panthenol | Panthenol | 0.50 |
| | Lara Care A-200 | Galactoarabinan | 0.25 |
| | | | |
| B | Baysiloneöl M 10 | Dimethicone | 1.00 |
| | Edeta BD | Disodium EDTA | 0.10 |
| | Copherol 1250 | Tocopheryl Acetate | 0.50 |
| | Cetiol OE | Dicaprylyl Ether | 3.00 |
| | Neo Heliopan® HMS | Homosalate | 5.00 |
| | Neo Heliopan® AV | Ethylhexyl Methoxycinnamate | 6.00 |
| | Neo Heliopan® 357 | Butyl Methoxydibenzoylmethane | 1.00 |
| | UV-Absorber gemäß Formel I | | 2.00 |
| | Alpha Bisabolol nat. H&R | Bisabolol | 0.10 |
| | Pemulen TR-2 | Acrylates/C10-30 Alkyl Acrylate Crosspolymer | 0.25 |
| | | | |

(fortgesetzt)

| Rezepturbeispiel 10 | | | |
|---|---|---|---|
| Sonnenschutzspray | | | |
| Teil | Rohstoffe | INCI Bezeichnung | % (Gew.) |
| C | Phenoxyethanol | Phenoxyethanol | 0.70 |
| | Solbrol M | Methylparaben | 0.20 |
| | Solbrol P | Propylparaben | 0.10 |
| | | | |
| D | NaOH, 10 %ig | Sodium Hydroxide | 0.60 |
| | | | |
| E | Parfümöl | Fragrance (Parfum) | 0.20 |

**Herstellungsverfahren**

Teil A: Lara Care A-200 unter Rühren in den anderen Bestandteilen von Teil A lösen.
Teil B: Alle Rohstoffe (ohne Pemulen) einwiegen und die kristallinen Substanzen unter Erwärmen lösen. Pemulen eindispergieren. Teil B zu Teil A geben und 1 Minute homogenisieren.
Teil C+D zugeben und nochmals 1-2 Minuten mit dem Ultra Turrax homogenisieren.

| Rezepturbeispiel 11 | | | |
|---|---|---|---|
| Sonnenschutz Hydrodispersionsgel (Balm) | | | |
| Teil | Rohstoffe | INCI Bezeichnung | % (Gew.) |
| A | Wasser, dest. | Water (Aqua) | 74.90 |
| | Carbopol 1342 | Acrylates/C10-30 Alkyl Acrylate Crosspolomer | 1.00 |
| | Triethanolamine | Triethanolamine | 1.20 |
| | | | |
| B | Neo Heliopan® Hydro, 30%ige Lösung neutralisiert mit TEA | Phenylbenzimidazole Sulfonic Acid | 10.00 |
| | | | |
| C | Neo Heliopan® AV | Ethylhexyl Methoxycinnamate | 3.00 |
| | UV-Absorber gemäß Formel I | | 2.00 |
| | Isopropylmyristat | Isopropyl Myristate | 4.00 |
| | Baysilone ÖL PK 20 | Phenyl Trimethicone | 3.00 |
| | Phenonip | Phenoxyethanol (and) Methylparaben (and) Butyparaben (and) Ethyparaben (and) Propylparaben | 0.50 |
| | Parfümöl | Parfum ( Fragrance) | 0.30 |
| | Bisabolol nat H&R | Bisabolol | 0.10 |

**Herstellungsverfahren**

Teil A: Carbopol in Wasser dispergieren und mit Natronlauge neutralisieren.
Teil B: Unter Rühren zu Teil A geben.
Teil C: Kristalline Bestandteile unter Erwärmen (max.40°C) in den anderen Rohstoffen von Teil C lösen und zu Teil A/B geben. Gut verrühren und anschließend homogenisieren (Homozenta)

| **Rezepturbeispiel 12** | | | |
|---|---|---|---|
| Hair Conditioner mit UV-Filtern | | | |
| Teil | Rohstoffe | INCI Bezeichnung | % (Gew.) |
| A | Emulgade 1000 NI | Cetearyl Alcohol (and) Ceteareth-20 | 2.00 |
| | Lanette 16 | Cetyl Alcohol | 1.00 |
| | Neo Heliopan® AV | 2 -Ethylhexyl Methoxycinnamate | 3.00 |
| | UV-Absorber gemäß Formel I | | 1.00 |
| | | | |
| B | Wasser, dest | Water (Aqua) | 91.70 |
| | Edeta BD | Disodium EDTA | 0.10 |
| | Phenonip | Phenoxyethanol (and) Methylparaben (and) Butyparaben (and) Ethyparaben (and) Propylparaben | 0.40 |
| | Dehyquart A-CA | Cetrimonium Chloride | 0.20 |
| | NaOH, 1%ig | Sodium Hydroxide | 0.30 |
| | | | |
| C | Parfümöl | Parfum ( Fragrance) | 0.30 |

**Herstellungsverfahren**

Teil A: Auf 80°C erhitzen.
Teil B: Auf 80°C erhitzen. Unter Rühren zu Teil A geben.
Teil C: Bei 40°C zugeben und auf RT abkühlen.

| **Rezepturbeispiel 13** | | | |
|---|---|---|---|
| Sonnenschutzlotion (O/W) | | | |
| Teil | Rohstoffe | INCI Bezeichnung | % (Gew.) |
| A | Tegin M | Glyceryl Stearate | 2.50 |
| | Tagat S | PEG-30 Glyceryl Stearate | 1.95 |
| | Lanette O | Cetearyl Alcohol | 2.20 |
| | Hallbrite TQ | Diethylhexylnaphthalate | 7,00 |
| | Cetiol B | Dibutyl Adipate | 5,00 |
| | Tegosoft TN | C12 - C15 Alkylbenzoate | 4.00 |
| | Myritol PC | Propylene Glycol Dicaprylate/Dicaprate | 4.00 |

(fortgesetzt)

| Rezepturbeispiel 13 | | | |
|---|---|---|---|
| Sonnenschutzlotion (O/W) | | | |
| Teil | Rohstoffe | INCI Bezeichnung | % (Gew.) |
| | Phenonip | Phenoxyethanol (and) Methylparaben (and) Butylparaben (and) Ethyl-paraben (and) Propylparaben | 0.15 |
| | UV-Absorber gemäß Formel I | | 2.00 |
| | Neo Heliopan® AV | Ethylhexyl Methoxycinnamate | 5.00 |
| | | | |
| B | Wasser, dest. | Water (Aqua) | 42,80 |
| | 1,2-Propylenglykol | Propylene Glycol | 2.00 |
| | Phenonip | Phenoxyethanol (and) Methylparaben (and) Butylparaben (and) Ethylparaben (and) Propylparaben | 0.30 |
| | | | |
| C | Wasser, dest. | Water (Aqua) | 19.00 |
| | Carbopol 2050 | Carbomer | 0.40 |
| | NaOH, 10%ig | Sodium Hydroxide | 1.70 |

**Herstellungsverfahren**

Teil A: Auf 80-85°C erhitzen.
Teil B: Auf 80-85°C erhitzen, unter Rühren Teil B zu Teil A geben.
Teil C: Carbopol in das Wasser eindispergieren und unter Rühren mit NaOH neutralisieren. Teil C bei ca. 60°C unter Rühren zugeben.

**Patentansprüche**

**1.** Indanylidenverbindungen der Formel

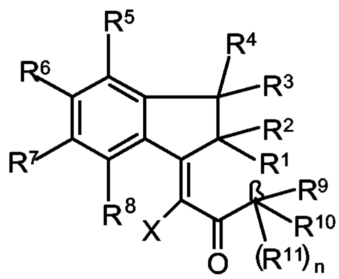

(I)

worin

$R^1$ bis $R^4$ unabhängig voneinander Wasserstoff, $C_1$-$C_{20}$-Alkyl oder $C_5$-$C_{10}$-Cycloalkyl bedeuten, mit der Maßgabe, dass auch zwei Substituenten an benachbarten C-Atomen zusammen eine gegebenenfalls substituierte $C_1$-$C_4$-Alkylengruppe bedeuten können;
weiterhin unabhängig voneinander $C_2$-$C_{20}$-Alkyl, in der mindestens eine Methylen-gruppe durch Sauerstoff, $C_3$-$C_{20}$-Alkenyl, $C_3$-$C_{20}$- Alkinyl oder einer Gruppe S ersetzt sein kann, bedeuten können,

wobei S eine Silan-, eine Oligosiloxan- oder eine Polysiloxan-Gruppe sein kann;

$R^5$ bis $R^8$ unabhängig voneinander Wasserstoff, $C_1$-$C_{20}$-Alkyl oder $C_5$-$C_{10}$-Cycloalkyl oder $C_1$ -$C_{20}$-Alkoxy, $C_5$-$C_{10}$-Cycloalkoxy, Hydroxy, Acetoxy, Acetamino, Carboxy, Carbalkoxy oder Carbamoyl bedeuten, zusätzlich zwei Substituenten von $R^5$ bis $R^8$ an benachbarten C-Atomen zusammen einen 5-7-gliedrigen Ring bilden können, der bis zu drei Heteroatome, insbesondere Sauerstoff oder Stickstoff enthält, wobei die Ringatome durch exocyclisch doppelt gebundenen Sauerstoff (Ketogruppe) substituiert sein können,
weiterhin im Fall von Alkoxy unabhängig voneinander $C_2$-$C_{20}$-Alkyl, in der mindestens eine Methylengruppe durch Sauerstoff, $C_3$-$C_{20}$-Alkenyl, $C_3$-$C_{20}$ Alkinyl oder einer Gruppe S ersetzt sein kann, bedeuten können,
wobei S eine Silan-, eine Oligosiloxan- oder eine Polysiloxan-Gruppe sein kann;

X für Cyano, $CON(R)_2$ oder $CO_2R$, wobei R für Wasserstoff oder $C_1$-$C_8$-Alkyl steht;

n für 1 oder 0 steht;

$R^9$ bis $R^{11}$ im Fall von n=1 Wasserstoff, $C_1$-$C_{20}$-Alkyl oder $C_5$-$C_{10}$-Cycloalkyl, Aryl oder Heteroaryl bedeuten können,
zusätzlich zwei Substituenten von $R^9$ bis $R^{11}$ gemeinsam mit dem β-Atom einen 3-7-gliedrigen Ring, der bis zu drei Heteroatome, insbesondere Sauerstoff oder Stickstoff enthalten kann, bilden können,
weiterhin im Fall von n=0 $R^9$ und $R^{10}$ gemeinsam mit dem β-Atom einen Aryl oder Heteroaryl-Rest bedeuten.

**2.** Indanylidenverbindungen nach Anspruch 1 der Formel

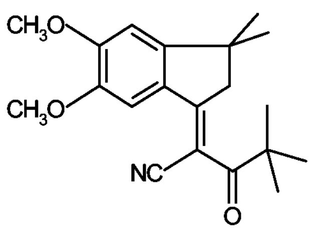
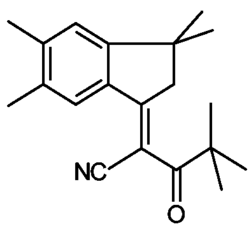
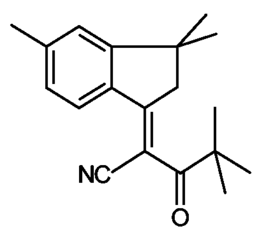

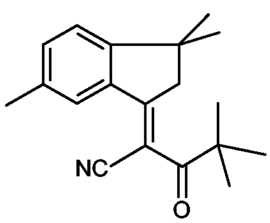
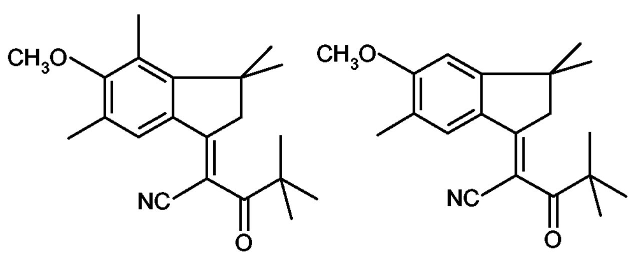

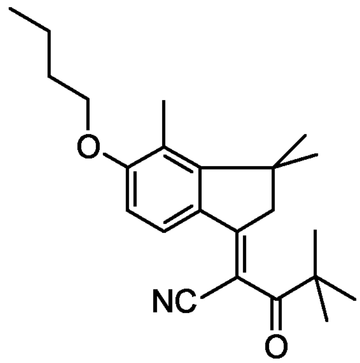
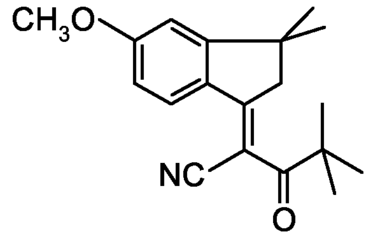

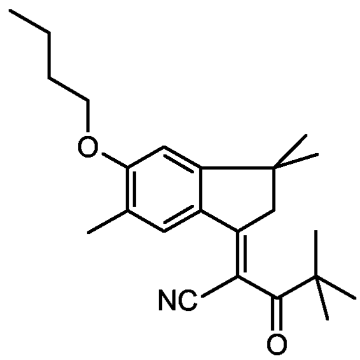
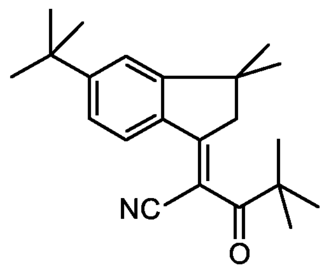

**3.** 2-(5,6-Dimethoxy-3,3-dimethyl-1-indanyliden)-4,4-dimethyl-3-oxo-pentansäurenitril, 2-(5-Methoxy-3,3,4,6-tetramethyl-1-indanyliden)-4,4-dimethyl-3-oxo-pentansäurenitril, 2-(3,3,5,6-tetramethyl-1-indanyliden)-4,4-dimethyl-3-oxo-pentansäurenitril, 2-(3,3,6-trimethyl-1-indanyliden)-4,4-dimethyl-3-oxopentansäurenitril, 2-(5,6-Ethylendioxo-3,3-dimethyl-1-indanyliden)-4,4-dimethyl-3-oxo-pentansäurenitril, 2-(5-Methoxy-3,3,6-trimethyl-1-indanyliden)-4,4-dimethyl-3-oxo-pentansäurenitril, 2-[(5-Methoxy-3,3-dimethyl-(2-methyl-3-(1,3,3,3-tetramethyl-1-(trimethyl-silyloxy)-disiloxanyl)-propyl)-indanyliden)]-4,4-dimethyl-3-oxo-pentansäurenitril und 2-(6-Acetoxy-3,3-dimethyl-5-methoxy-1-indanyliden)-4,4-dimethyl-3-oxo-pentan-säurenitril.

**4.** Kosmetische Mittel zum Schutz von Haut und Haaren enthaltend Indanylidenverbindungen der Formel

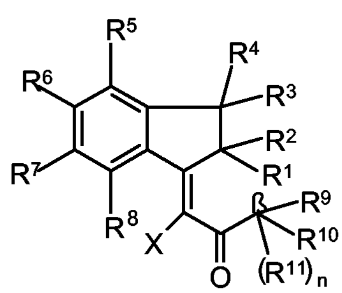

(I)

worin

$R^1$ bis $R^4$ unabhängig voneinander Wasserstoff, $C_1$-$C_{20}$-Alkyl oder $C_5$-$C_{10}$-Cycloalkyl bedeuten, mit der Maßgabe, dass auch zwei Substituenten an benachbarten C-Atomen zusammen eine gegebenenfalls substituierte $C_1$-$C_4$-Alkylengruppe bedeuten können;
weiterhin unabhängig voneinander $C_2$-$C_{20}$-Alkyl, in der mindestens eine Methylen-gruppe durch Sauerstoff, $C_3$-$C_{20}$-Alkenyl, $C_3$-$C_{20}$- Alkinyl oder einer Gruppe S ersetzt sein kann, bedeuten können,

wobei S eine Silan-, eine Oligosiloxan- oder eine Polysiloxan-Gruppe sein kann;

$R^5$ bis $R^8$ unabhängig voneinander Wasserstoff, $C_1$-$C_{20}$-Alkyl oder $C_5$-$C_{10}$-Cycloalkyl oder $C_1$ -$C_{20}$-Alkoxy, $C_5$-$C_{10}$-Cycloalkoxy, Hydroxy, Acetoxy, Acetamino, Carboxy, Carbalkoxy oder Carbamoyl bedeuten, zusätzlich zwei Substituenten von $R^5$ bis $R^8$ an benachbarten C-Atomen zusammen einen 5-7-gliedrigen Ring bilden können, der bis zu drei Heteroatome, insbesondere Sauerstoff oder Stickstoff enthält, wobei die Ringatome durch exocyclisch doppelt gebundenen Sauerstoff (Ketogruppe) substituiert sein können,
weiterhin im Fall von Alkoxy unabhängig voneinander $C_2$-$C_{20}$-Alkyl, in der mindestens eine Methylengruppe durch Sauerstoff, $C_3$-$C_{20}$-Alkenyl, $C_3$-$C_{20}$ Alkinyl oder einer Gruppe S ersetzt sein kann, bedeuten können,
wobei S eine Silan-, eine Oligosiloxan- oder eine Polysiloxan-Gruppe sein kann;

X für Cyano, $CON(R)_2$ oder $CO_2R$ wobei R für Wasserstoff oder $C_1$-$C_8$-Alkyl steht;

n für 1 oder 0 steht;

$R^9$ bis $R^{11}$ im Fall von n=1 Wasserstoff, $C_1$-$C_{20}$-Alkyl oder $C_5$-$C_{10}$-Cycloalkyl, Aryl oder Heteroaryl bedeuten können,
zusätzlich zwei Substituenten von $R^9$ bis $R^{11}$ gemeinsam mit dem β-Atom einen 3-7-gliedrigen Ring, der bis zu drei Heteroatome, insbesondere Sauerstoff oder Stickstoff enthalten kann, bilden können,
weiterhin im Fall von n=0 $R^9$ und $R^{10}$ gemeinsam mit dem β-Atom einen Aryl oder Heteroaryl-Rest bedeuten.

**5.** Kosmetische Mittel zum Schutz von Haut und Haaren nach Anspruch 4, enthaltend

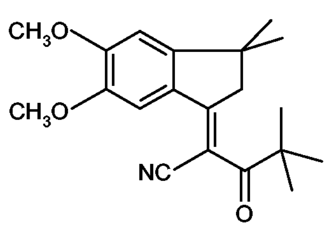
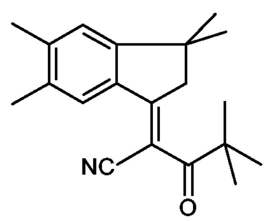
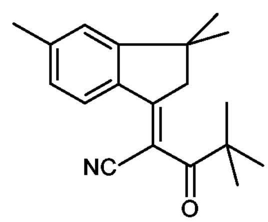

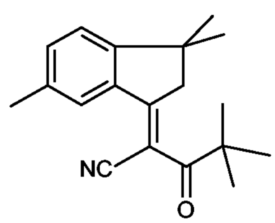
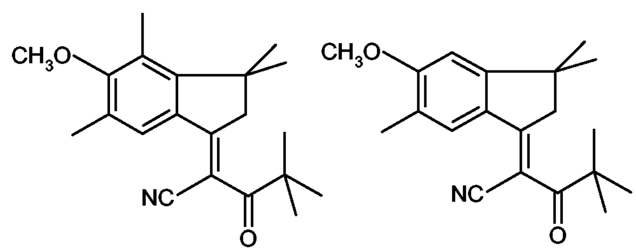

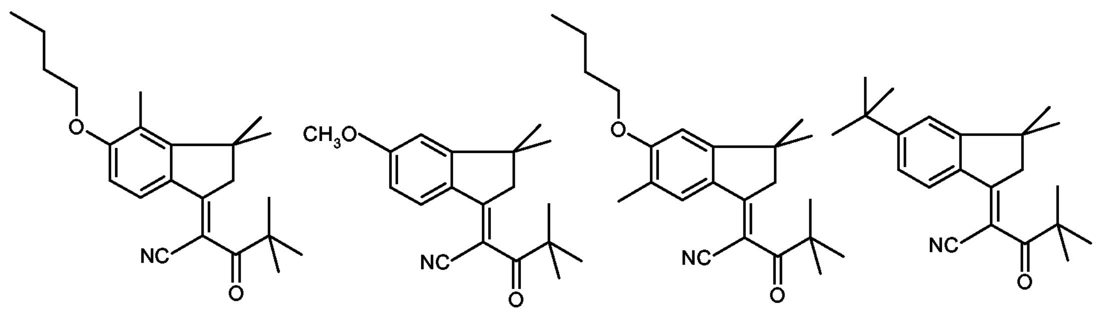

6. Kosmetische Mittel zum Schutz von Haut und Haaren nach Anspruch 4, enthaltend 2-(5,6-Dimethoxy-3,3-dimethyl-1-indanyliden)-4,4-dimethyl-3-oxopentansäurenitril, 2-(5-Methoxy-3,3,4,6-tetramethyl-1-indanyliden)-4,4-dimethyl-3-oxo-pentansäurenitril, 2-(3,3,5,6-tetramethyl-1-indanyliden)-4,4-dimethyl-3-oxo-pentansäurenitril, 2-(3,3,6-trimethyl-1-indanyliden)-4,4-dimethyl-3-oxo-pentansäurenitril, 2-(5,6-Ethylendioxo-3,3-dimethyl-1-indanyliden)-4,4-dimethyl-3-oxo-pentansäurenitril, 2-(5-Methoxy-3,3,6-trimethyl-1-indanyliden)-4,4-dimethyl-3-oxo-pentansäurenitril, 2-[(5-Methoxy-3,3-dimethyl-(2-methyl-3-(1,3,3,3-tetramethyl-1-(trimethyl-silyloxy)-disiloxanyl)-propyl)-indanyliden)]-4,4-dimethyl-3-oxo-pentan-säurenitril oder 2-(6-Acetoxy-3,3-dimethyl-5-methoxy-1-indanyliden)-4,4-dimethyl-3-oxopentansäurenitril.

7. Verwendung von Indanylidenverbindungen nach den Ansprüchen 1 bis 3 als UV-Absorber in kosmetischen Mitteln zum Schutz für Haut und/oder der Haare gegen UV-Strahlung.

8. Verwendung von Indanylidenverbindungen nach den Ansprüchen 1 bis 3 als UV-Absorber in Kombination mit UV-Absorbern des Typs Methoxycinnamat-Derivate oder des Typs Dibenzoylmethan-Derivate.

9. Verwendung von Indanylidenverbindungen nach den Ansprüchen 1 bis 3 zur Photostabilisierung von UV -Absorbern des Typs Dibenzoylmethan-Derivate und Methoxycinnamate.

10. Verwendung von Indanylidenverbindungen nach den Ansprüchen 1 bis 3 als UV-Absorber in Kombination mit einen oder mehreren UV-A-, UV-Bund/oder Breitbandfiltern als Einzelsubstanzen oder in Gemischen untereinander, in der Lipidphase und/oder in der wässrigen Phase.

11. Verwendung von Indanylidenverbindungen nach den Ansprüchen 1 bis 3 zur Photostabilisierung von Kombinationen von UV-Absorbern des Typs der Methoxycinnamat-Derivate mit Dibenzoylmethan-Derivaten.

12. Kosmetische Mittel nach Anspruch 4, **dadurch gekennzeichnet, dass** sie außerdem mindestens ein Bräunungs- und/oder künstliches Selbstbräunungsmittel für die Haut enthalten.

13. Kosmetische Mittel nach Anspruch 4 , **dadurch gekennzeichnet, dass** sie neben den erfindungsgemäßen Verbindungen der Formel I einen oder mehrere weitere UV-Absorber enthalten.

14. Kosmetische Mittel nach Anspruch 4 , **dadurch gekennzeichnet, daß** sie neben den erfindungsgemäßen Verbindungen der Formel I einen oder mehrere weitere UV-Absorber aus der Reihe Ethylhexylmethoxycinnamat, 2-Ethylhexyl-2-cyano-3,3-diphenylacrylat, 2-Phenylbenzimidazolsulfonsäure, Methylbenzylidencampher, 4-t-Butyl-4'-methoxy-dibenzoylmethan, Phenylen-bis-benzimidazyl-tetrasulfonsäure-dinatriumsalz, Terephthalyliden-dibomansulfonsäure und Salze, Phenol, 2-(2H-benzotriazol-2-yl)-4-methyl-6-(2-methyl-3(1,3,3,3-tetramethyl-1-(trimethylsilyl)-oxy)-disiloxyanyl)-propyl), Benzylidenmalonat-Polysiloxan, 2,2'-Methylen-bis-(6-(2H-benztriazol-2-yl)-4-1,1,3,3-tetramethylbutyl)-phenol), 2,4-Bis-[{(4-(2-Ethyl-hexyloxy-2-hydroxy}-phenyl]-6-(4-methoxyphenyl)-1,3,5-triazin, 4,4',4-(1,3,5-Triazin-2,4,6-triyltriimino)-tris-benzoesäure-tris(2-ethylhexylester), 4,4'-[(6-[4-(1,1-Dimethyl)-aminocarbonyl)-phenylamino]-1,3,5-triazin-2,4-diyl)diimino]-bis-(benzoesäure-2-ethylhexylester), Zinkoxid und Titandioxid enthalten.

15. Kosmetische Mittel nach Anspruch 4, **dadurch gekennzeichnet, dass** sie weitere UV-Absorber und zusätzlich umhüllte oder nicht umhüllte Pigmente oder Nanopigmente von Metalloxiden enthalten.

**16.** Kosmetische Mittel nach Anspruch 4 , **dadurch gekennzeichnet, dass** sie weitere UV-Absorber und zusätzlich feinteilige Pigmente enthalten, so dass daraus eine UV-Breitbandschutzleistung mit einer kritischen Wellenlänge $\lambda_{crit.}$> 380 nm resultiert.

Europäisches Patentamt

# EUROPÄISCHER RECHERCHENBERICHT

Nummer der Anmeldung
EP 04 10 2032

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,A | EP 0 823 418 A (HAARMANN & REIMER GMBH) 11. Februar 1998 (1998-02-11)<br>* das ganze Dokument * | 1-16 | C07C255/40<br>C07D319/18<br>C07F7/08<br>G03C1/815<br>A61K7/42 |
| X | WO 00/14172 A (MICHAELIS STEPHAN ;WOMELSDORF HERMANN JENS (DE); BERNETH HORST (DE) 16. März 2000 (2000-03-16)<br>siehe Seite 60 Beispiel 1-9: "Gelbfilter"<br>* Anspruch 1 * | 1-3 | |
| X | DATABASE CA [Online]<br>CHEMICAL ABSTRACTS SERVICE, COLUMBUS, OHIO, US;<br>CHIACCHIO, UGO ET AL: "Novel approach to the ring-opening of 4-isoxazolines: one-pot synthesis of.alpha.,.beta.-enones"<br>XP002190251<br>gefunden im STN<br>Database accession no. 116:128741 CA<br>siehe RN:139286-80-9 [Marker: 00055]<br>* Zusammenfassung *<br>& TETRAHEDRON,<br>Bd. 48, Nr. 1, 1992, Seiten 123-132, | 1 | |

RECHERCHIERTE SACHGEBIETE (Int.Cl.7)
C07C
C07D
C07F
G03C
A61K

Der vorliegende Recherchenbericht wurde für alle Patentansprüche erstellt

| Recherchenort | Abschlußdatum der Recherche | Prüfer |
|---|---|---|
| München | 13. August 2004 | Goetz, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 04 10 2032

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-08-2004

| Im Recherchenbericht angeführtes Patentdokument | Datum der Veröffentlichung | Mitglied(er) der Patentfamilie | Datum der Veröffentlichung |
|---|---|---|---|
| EP 0823418 A | 11-02-1998 | DE 19631863 A1 | 12-02-1998 |
| | | AU 738152 B2 | 13-09-2001 |
| | | AU 3244797 A | 12-02-1998 |
| | | CA 2212256 A1 | 07-02-1998 |
| | | DE 59708417 D1 | 14-11-2002 |
| | | EP 0823418 A2 | 11-02-1998 |
| | | ES 2187704 T3 | 16-06-2003 |
| | | JP 10077220 A | 24-03-1998 |
| | | US 6153175 A | 28-11-2000 |
| | | US 5965066 A | 12-10-1999 |
| | | ZA 9706991 A | 03-03-1998 |
| WO 0014172 A | 16-03-2000 | DE 19840938 A1 | 09-03-2000 |
| | | DE 19920360 A1 | 11-01-2001 |
| | | AU 747888 B2 | 30-05-2002 |
| | | AU 5855299 A | 27-03-2000 |
| | | WO 0014172 A1 | 16-03-2000 |
| | | EP 1114117 A1 | 11-07-2001 |
| | | JP 2002524762 T | 06-08-2002 |
| | | US 6600589 B1 | 29-07-2003 |

EPO FORM P0461

Für nähere Einzelheiten zu diesem Anhang : siehe Amtsblatt des Europäischen Patentamts, Nr.12/82